(19) Europäisches Patentamt
European Patent Office
Office européen des brevets

(11) **EP 4 732 839 A1**

(12) **EUROPEAN PATENT APPLICATION**
published in accordance with Art. 153(4) EPC

(43) Date of publication:
**29.04.2026 Bulletin 2026/18**

(21) Application number: **24855663.1**

(22) Date of filing: **13.08.2024**

(51) International Patent Classification (IPC):
***A61K 31/519*** *(2006.01)* ***A61K 31/5377*** *(2006.01)*
***A61P 31/12*** *(2006.01)* ***A61P 35/00*** *(2006.01)*

(52) Cooperative Patent Classification (CPC):
**A61K 9/00; A61K 31/519; A61K 31/5377; A61K 45/06; A61P 1/16; A61P 31/12; A61P 31/14; A61P 31/20; A61P 35/00; A61P 35/04**

(86) International application number:
**PCT/CN2024/111818**

(87) International publication number:
**WO 2025/039937 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.08.2023 CN 202311056883**

(71) Applicant: **Beijing Synthetic Vaccine Biosciences Co., Ltd**
**Beijing 102206 (CN)**

(72) Inventors:
- **LIAO, Xuebin**
  **Beijing 102206 (CN)**
- **WANG, Zhisong**
  **Beijing 102206 (CN)**
- **WANG, Liangliang**
  **Beijing 102206 (CN)**

(74) Representative: **HGF**
**HGF Limited**
**4th Floor, 1 City Square**
**Leeds LS1 2ES (GB)**

# (54) USE OF COMPOUND IN TREATMENT OF LIVER DISEASES

(57) Use of a compound in the treatment of liver diseases. Experimental results show that the compound has a relatively high liver-tissue-targeting property, very low toxicity to liver primary cells and high liver safety, has significant efficacy on liver diseases such as viral hepatitis (e.g., hepatitis B) and liver cancers (including primary liver cancers and metastatic liver cancers), and has relatively good physicochemical properties, metabolic stability, bioavailability and safety, thus having very good drug development prospects.

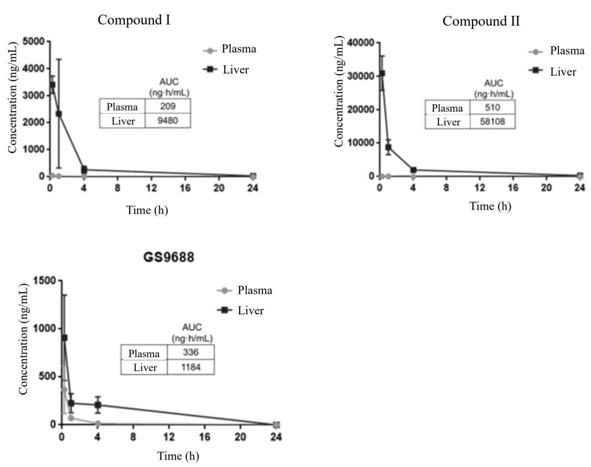


FIG. 1



EP 4 732 839 A1

## Description

### TECHNICAL FIELD

The present invention relates to the technical field of chemical pharmaceutics, and in particular to use of a compound in treating liver diseases.

### BACKGROUND

The liver is an organ in the body that mainly performs metabolic functions and plays roles such as deoxidation, storage of glycogen, and synthesis of secretory proteins in the body. The liver also produces bile in the digestive system. The liver is the largest internal organ of the human body, located in the abdominal region, beneath the right diaphragm, anterior to the gallbladder, in front of the right kidney, and above the stomach. The liver is the largest digestive gland of the human digestive system. The average weight of the liver of an adult is about 1.5 kg, and the liver is a red-brown V-shaped organ. The liver is the major organ for urea synthesis and is also an important organ for metabolism.

Liver diseases are diseases with high morbidity and mortality. Liver diseases may be classified into viral and non-viral types. Hepatitis viruses are the major pathogens that cause liver diseases. The currently identified hepatitis viruses include hepatitis A, hepatitis B, hepatitis C, hepatitis D, hepatitis E viruses, and the like. Non-viral liver diseases are liver injuries caused by drugs, toxins, alcohol, and other factors. The development of liver diseases generally includes several stages, including acute hepatitis, chronic hepatitis, hepatic fibrosis, cirrhosis (hepatic ascites), and hepatocellular carcinoma. In addition, the anatomical characteristics of the liver are the dual blood supply from the hepatic artery and the portal vein, such that malignant tumors are prone to liver metastasis. Primary lesions of liver metastatic cancer may originate from multiple organs throughout the body, mainly digestive tract tumors, which account for about 35% to 50% of liver metastatic cancer.

Currently, few therapeutic drugs are approved for treating advanced hepatocellular carcinoma, mainly the kinase inhibitor sorafenib and the immune checkpoint blocking agent PD-1/PD-L1 monoclonal antibody. Sorafenib is a first-line therapeutic agent for liver cancer; however, according to reports, its clinical efficacy is limited and it can easily cause adverse reactions. According to the results of the phase III clinical trial (SHARP trial) of sorafenib, the median survival of patients with advanced unresectable HCC (N = 602) treated with sorafenib was 10.7 months, compared with 7.9 months in the placebo group, indicating that the improvement in survival was not significant. The progression-free survival with sorafenib was 167 days, compared with 84 days in the placebo group. According to the CheckMate 459 clinical trial, which is a phase III clinical trial to compare nivolumab with sorafenib for first-line treatment of advanced hepatocellular carcinoma (aHCC), the administration regimen was nivolumab at 240 mg (intravenous drip, once every 2 weeks) and sorafenib at 400 mg (oral administration, twice daily). The results were published at the 2019 European Society for Medical Oncology (ESMO) Annual Meeting: the mOS was 16.4 months for the nivolumab group (371 cases) and 14.7 months for the sorafenib group (372 cases) (HR = 0.85, 95% CI: 0.72-1.02, P = 0.075 2), which did not reach the predefined threshold for statistical significance, and the ORRs of the two groups were 15% and 7%, respectively. The phase III clinical trial did not reach the expected primary research endpoint (OS), but the efficacy of nivolumab was indeed improved compared with that of sorafenib. Currently, a plurality of immune checkpoint blocking antibodies have been approved for marketing for liver cancer treatment. However, to prolong the survival and biochemical quality of patients with liver cancer, there is an urgent need to develop novel targeted immunotherapeutic drugs for preventing and treating liver diseases.

### SUMMARY

To overcome the defects in the prior art, the present invention provides use of a small molecule compound having an immunomodulatory function in the prevention and/or treatment of liver diseases.

In a first aspect of the present invention, provided is use of a compound or a pharmaceutically acceptable salt thereof in preparing a medicament for preventing and/or treating a liver disease, wherein the compound has the following structure:

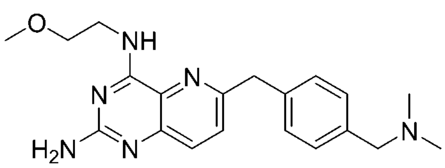

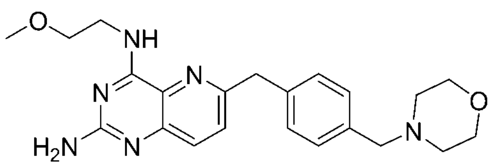

.

Specifically, the liver disease is a disease occurring in the liver, e.g., an infectious disease, a neoplastic disease, a vascular disease, a metabolic disease, a toxic disease, an autoimmune disease, a genetic disease, or hepatolithiasis. In one embodiment of the present invention, the liver disease is hepatitis, e.g., viral hepatitis, alcoholic hepatitis, steatohepatitis, drug-induced hepatitis, or autoimmune hepatitis, particularly viral hepatitis.

Specifically, the viral hepatitis includes: hepatitis A, hepatitis B, hepatitis C, hepatitis D, hepatitis E, and hepatitis G, particularly hepatitis B.

In one embodiment of the present invention, the liver disease is a liver tumor, e.g., hepatic hemangioma, hepatic adenoma, hepatic lipoma, non-parasitic cyst of liver, focal nodular hyperplasia of liver, liver cancer, bile duct cancer, hepatoblastoma, or hepatic sarcoma, particularly liver cancer.

Specifically, the liver cancer is primary liver cancer or metastatic liver cancer.

Specifically, a primary tumor of the metastatic liver cancer may be colorectal cancer, colon cancer, gastric cancer, pancreatic cancer, gastric and intestinal leiomyosarcoma, lung cancer, breast cancer, renal cancer, cervical cancer, ovarian cancer, prostate cancer, or head and neck tumor. In one embodiment of the present invention, the metastatic liver cancer is colon cancer metastatic liver cancer.

In one embodiment of the present invention, the liver disease is liver injury, e.g., hepatic fibrosis or cirrhosis.

In one embodiment of the present invention, the liver disease is fatty liver.

In one embodiment of the present invention, the liver disease is hepatolithiasis.

Specifically, in the use, the compound or the pharmaceutically acceptable salt thereof may be used alone or in combination with other type of active ingredient (a second active ingredient) (the compound or the pharmaceutically acceptable salt thereof and the second active ingredient may be in the same dosage form or in different dosage forms, and the two may be administered simultaneously, separately, or sequentially).

Specifically, for the embodiment in which the liver disease is a liver tumor, the second active ingredient may be one or more of a radiotherapeutic agent, a chemotherapeutic agent, an immunotherapeutic agent, an antiangiogenic agent, a cytokine, a hormone, a polynucleotide, an antibody, an immunologically active fragment, an immune checkpoint blocking agent, a cell therapy drug, a small nucleic acid drug, an mRNA vaccine, a tumor vaccine, and the like.

Specifically, the immune checkpoint may be, for example, PD-1, PD-L1, CTLA-4, BTLA, TIM-3, LAG-3, TIGIT, LAIR1, 2B4, or CD160, particularly PD-1 or PD-L1.

Specifically, the immune checkpoint inhibitor may be an antibody, a small molecule compound, or a combination thereof.

In one embodiment of the present invention, the second active ingredient is a PD-1 antibody, e.g., pembrolizumab, nivolumab, cemiplimab, nofazinlimab (CS1003), tislelizumab, toripalimab, sintilimab, camrelizumab, penpulimab, zimberelimab, or serplulimab.

In one embodiment of the present invention, the second active ingredient is a PD-L1 antibody, e.g., atezolizumab, duvalumab, durvaluamb, sugemalimab, or envafolimab.

Other PD-1/PD-L1 monoclonal antibodies under development further include, for example, penpulimab (Akeso Biopharma), HLX-10 (Henlius), cemiplimab (Sanofi), SCT-I10A (SinoCellTech), AMG404 (Amgen), TY101 (Dayou Biotech), sasanlimab (Pfizer), genolimzumab (Genor Biopharma), LZM009 (Livzon), HX008 (Lepu Medical), LVHN6051 (Lyvgen Biopharma), F520 (Lunan Pharmaceutical), spartalizumab (Novartis), QL1604 (Qilu Pharmaceutical), 609A (Sunshine Guojian Pharmaceutical), SG001 (Sumgen Bio), STW204mAb (Stainwei), zimberelimab (WuXi Biologics), and retifanlimab (Zai Lab).

In one embodiment of the present invention, the second active ingredient is a CTLA-4 antibody, e.g., tremelimumab or ipilimumab.

Specifically, the chemotherapeutic agent is, for example, but is not limited to, chlorambucil, melphalan, cyclophosphamide, ifosfamide, busulfan, carmustine, lomustine, streptozotocin, cisplatin, carboplatin, oxaliplatin, dacarbazine, temozolomide, procarbazine, methotrexate, fluorouracil, cytarabine, gemcitabine, capecitabine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunomycin, mitoxantrone, bleomycin, mitomycin, ixabepilone, tamoxifen, flutamide, gonadorelin analog, megestrol, prednisone, dexamethasone, methylprednisolone, thalidomide, interferon alpha, calcium folinate, sirolimus, sirolimus lipidation, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib, motesanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, tasocitinib, telatinib, tivantinib, tivozanib, tofacitinib, trametinib, vandetanib, veliparib, or a combination thereof.

In one embodiment of the present invention, the chemotherapeutic agent is a multi-target receptor tyrosine kinase (RTK) inhibitor, e.g., imatinib, sorafenib, sunitinib, dasatinib, lapatinib, pazotinib, crizotinib, vandetanib, regorafenib, cabozantinib, axitinib, ponatinib, radotinib, afatinib, nintedanib, lenvatinib, neratinib, or lenvatinib, particularly sorafenib or lenvatinib.

More specifically, the chemotherapeutic agent is selected from: fluorouracil, doxorubicin, cisplatin, mitomycin, gemcitabine, capecitabine, irinotecan, sorafenib, sunitinib, erlotinib, and lenvatinib, particularly sorafenib and lenvatinib.

Specifically, the antibody includes, but is not limited to: PRO542, which is an anti-HIV gp120 antibody fused to CD4 (Progenics/Genzyme Transgenics); MDX-010 (Medarex, New Jersey), which is a humanized anti-CTLA-4 antibody; SYNAGIS (MedImmune, Maryland), which is a humanized anti-respiratory syncytial virus (RSV) monoclonal antibody; HERCEPTIN (trastuzumab) (Genentech, California), which is a humanized anti-HER2 monoclonal antibody; Humanized anti-CD18 F(ab')2 (Genentech); CDP860, which is a humanized anti-CD18 F(ab')2 (Celltech, UK); Ostavir, which is a human anti-hepatitis B virus antibody (Protein Design Lab/Novartis); PROTOVIRTM, which is a humanized anti-CMV IgG1 antibody (Protein Design Lab/Novartis); MAK-195 (SEGARD), which is a murine anti-TNF-$\alpha$ F(ab')2 (Knoll Pharma/BASF); IC14, which is an anti-CD14 antibody (ICOSPharm); Humanized anti-VEGF IgG1 antibody (Genentech); OVAREXTM, which is a murine anti-CA125 antibody (Altarex); PANOREXTM, which is a murine anti-17-IA cell surface antigen IgG2a antibody (GlaxoWellcome/Centocor); BEC2, which is a murine anti-idiotype (GD3 epitope) IgG antibody (ImClone System); IMC-C225, which is a chimeric anti-EGFR IgG antibody (ImClone System); VitaxinTM, which is a humanized anti-$\alpha V\beta 3$ integrin antibody (Applied Molecular Evolution/MedImmune); Campath-1H/LDP-03, which is a humanized anti-CD52 IgG1 antibody (LeukoSite); SmartM195, which is a humanized anti-CD33 IgG antibody (Protein Design Lab/Kanebo); RITUXANTM (RituximabTM), which is a chimeric anti-CD20 IgG1 antibody (IDEC Pharm/Genentech, Roche/Zettyaku); LYMPHOCIDETM, which is a humanized anti-CD22 IgG antibody (Immunomedics); SmartID10, which is a humanized anti-HLA antibody (Protein Design Lab); ONCOLYMTM (Lym-1), which is a radiolabeled murine anti-HLADR antibody (Techniclone); ABX-IL8, which is a human anti-IL8 antibody (Abgenix); Anti-CD11a, which is a humanized IgG1 antibody (Genentech/Xoma); ICM3, which is a humanized anti-ICAM3 antibody (ICOS); IDEC-114, which is a primatized anti-CD80 antibody (IDEC Pharm/Mitsubishi); ZEVALINTM, which is a radiolabeled murine anti-CD20 antibody (IDEC/ScheringAG); IDEC-131, which is a humanized anti-CD40L antibody (IDEC/Eisai); IDEC-151, which is a primatized anti-CD4 antibody (IDEC); IDEC-152, which is a primatized anti-CD23 antibody (IDEC/Seikagaku); SMART anti-CD3, which is a humanized anti-CD3 IgG (Protein Design Lab); SG1.1, which is a humanized anti-complement factor 5 (C5) antibody (Alexion Pharm); D2E7, which is a humanized anti-TNF-$\alpha$ antibody (CAT/BASF); CDP870, which is a humanized anti-TNF-$\alpha$ Fab fragment (Celltech); IDEC-151, which is a primatized anti-CD4 IgG1 antibody (IDEC Pharm/SmithKline Beecham); MDX-CD4, which is a human anti-CD4 IgG antibody (Medarex/Eisai/-Genmab); CDP571, which is a humanized anti-TNF-$\alpha$ IgG4 antibody (Celltech); LDP-02, which is a humanized anti-$\alpha 4\beta 7$ antibody (LeukoSite/Genentech); OrthoCloneOKT4A, which is a humanized anti-CD4 IgG antibody (OrthoBiotech); ANTOVATM, which is a humanized anti-CD40L IgG antibody (Biogen); ANTEGRENTM, which is a humanized anti-VLA-4 IgG antibody (Elan); MDX-33, which is a human anti-CD64 (Fc$\gamma$R) antibody (Medarex/Centeon); SCH55700, which is a humanized anti-IL-5 IgG4 antibody (Celltech/ScheringAG); SB-240563 and SB-240683, which are humanized anti-IL-5 and humanized anti-IL-4 antibodies, respectively (SmithKline Beecham); rhuMab-E25, which is a humanized anti-IgE IgG1 antibody (Genentech/Novartis/Tanox Biosystems); ABX-CBL, which is a murine anti-CD-147 IgM antibody (Abgenix); BTI-322, which is a rat anti-CD2 IgG antibody (MedImmune/BioTransplant); Orthoclone/OKT3, which is a murine anti-CD3 IgG2a antibody (OrthoBiotech); SIMULECTTM, which is a chimeric anti-CD25 IgG1 antibody (Novartis Pharma); LDP-01, which is a humanized anti-$\beta 2$-integrin IgG antibody (LeukoSite); Anti-LFA-1, which is a murine anti-CD18 F(ab')2 (Pasteur-Merieux/Immunotech); CAT-152, which is a human anti-TGF-$\beta 2$ antibody (CambridgeAbTech); CorsevinM, which is a chimeric anti-Factor VII antibody (Centocor); MDX-1106, which is a PD-1 antibody (Bristol Myers Squibb); and MDX-1105, which is a PD-L1 antibody (Roche).

Specifically, the medicament further comprises one or more pharmaceutically acceptable auxiliary materials, such as, one or more of a disintegrant, a binder, a lubricant, a suspending agent, a stabilizer, a filler, an absorption promoter, a surfactant, a flavoring agent, an antioxidant, a preservative, isotonic sterile saline (sodium dihydrogen phosphate, disodium hydrogen phosphate, sodium chloride, potassium chloride, calcium chloride, magnesium chloride, or the like, or a mixture of the salts described above), and the like.

In one embodiment of the present invention, the medicament further comprises one or more pharmaceutically acceptable auxiliary materials in an oral dosage form.

Specifically, the medicament may be administered by any suitable route of administration, such as gastrointestinal or parenteral routes (e.g., intravenous, intramuscular, subcutaneous, intraorgan, intranasal, intradermal, instillation, intracerebral, or intrarectal administrations).

Specifically, the medicament may be in any suitable dosage form, such as dosage forms for gastrointestinal administration, for example, but not limited to, tablets, pills, powders, granules, capsules, lozenges, syrups, liquids, emulsions, or suspensions; dosage forms for parenteral administration, for example, dosage forms for injection administration such as injections (e.g., for subcutaneous, intravenous, intramuscular, or intraperitoneal injections), dosage forms for respiratory administration such as sprays, aerosols, or powder aerosols, dosage forms for skin administration such as external solutions, lotions, ointments, plasters, pastes, or patches, dosage forms for mucosa administration such as eye drops, eye ointments, nose drops, gargles, or sublingual tablets, or dosage forms for cavity administration such as suppositories, aerosols, effervescent tablets, drops, or dripping pills, which are used for rectum, vagina, urethra, nasal

cavity, auditory canal, or the like.

In one embodiment of the present invention, the medicament is in an oral dosage form.

In one embodiment of the present invention, the medicament further comprises a second active ingredient, wherein the compound is contained in an oral dosage form, and the second active ingredient is contained in an injection dosage form.

In a second aspect of the present invention, provided is a pharmaceutical composition comprising a compound or a pharmaceutically acceptable salt thereof and a second active ingredient (as described in the first aspect of the present invention), and, optionally, one or more pharmaceutically acceptable auxiliary materials. The compound has the following structure:

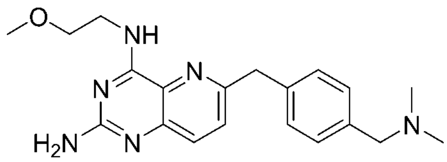

I

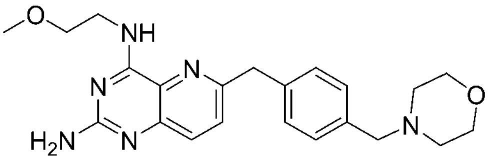

II

.

In one embodiment of the present invention, the second active ingredient is an immune checkpoint inhibitor, such as a PD-1 antibody, e.g., pembrolizumab, nivolumab, cemiplimab, nofazinlimab (CS1003), tislelizumab, toripalimab, sintilimab, camrelizumab, penpulimab, zimberelimab, or serplulimab; a PD-L1 antibody, e.g., atezolizumab, duvalumab, durvaluamb, sugemalimab, or envafolimab; a CTLA-4 antibody, e.g., tremelimumab or ipilimumab; or other PD-1/PD-L1 monoclonal antibodies under development, e.g., penpulimab (Akeso Biopharma), HLX-10 (Henlius), cemiplimab (Sanofi), SCT-I10A (SinoCellTech), AMG404 (Amgen), TY101 (Dayou Biotech), sasanlimab (Pfizer), genolimzumab (Genor Biopharma), LZM009 (Livzon), HX008 (Lepu Medical), LVHN6051 (Lyvgen Biopharma), F520 (Lunan Pharmaceutical), spartalizumab (Novartis), QL1604 (Qilu Pharmaceutical), 609A (Sunshine Guojian Pharmaceutical), SG001 (Sumgen Bio), STW204mAb (Stainwei), zimberelimab (WuXi Biologics), or retifanlimab (Zai Lab).

In one embodiment of the present invention, the pharmaceutical composition comprises the compound (particularly compound II) or the pharmaceutically acceptable salt thereof and the PD-1 antibody, and, optionally, one or more pharmaceutically acceptable auxiliary materials. Specifically, the pharmaceutical composition comprises an oral dosage form and an injection dosage form.

In one embodiment of the present invention, the pharmaceutical composition comprises compound I or the pharmaceutically acceptable salt thereof and nofazinlimab (CS1003), and, optionally, one or more pharmaceutically acceptable auxiliary materials. Specifically, the pharmaceutical composition comprises an oral dosage form and an injection dosage form.

In one embodiment of the present invention, the pharmaceutical composition comprises compound II or the pharmaceutically acceptable salt thereof and nofazinlimab (CS1003), and, optionally, one or more pharmaceutically acceptable auxiliary materials. Specifically, the pharmaceutical composition comprises an oral dosage form and an injection dosage form.

In one embodiment of the present invention, the second active ingredient is a chemotherapeutic agent, as described in the first aspect of the present invention, e.g., fluorouracil, doxorubicin, cisplatin, mitomycin, gemcitabine, capecitabine, irinotecan, sorafenib, sunitinib, lenvatinib, or erlotinib, particularly sorafenib and lenvatinib.

In one embodiment of the present invention, the pharmaceutical composition comprises the compound (particularly compound II) or the pharmaceutically acceptable salt thereof and sorafenib, and optionally, one or more pharmaceutically acceptable auxiliary materials. Specifically, the pharmaceutical composition is in an oral dosage form.

In a third aspect of the present invention, provided is use of a combination of a compound or a pharmaceutically acceptable salt thereof and a second active ingredient in preparing a medicament for preventing and/or treating a liver disease.

Specifically, the compound and the second active ingredient are as described in the first aspect of the present invention.

Specifically, the liver disease is as described in the first aspect of the present invention. In one embodiment of the present invention, the liver disease is a liver tumor, e.g., hepatic hemangioma, hepatic adenoma, hepatic lipoma, non-parasitic cyst of liver, focal nodular hyperplasia of liver, liver cancer, bile duct cancer, hepatoblastoma, or hepatic sarcoma, particularly liver cancer, which may be primary liver cancer or metastatic liver cancer. Specifically, a primary tumor of the metastatic liver cancer may be colorectal cancer, colon cancer, gastric cancer, pancreatic cancer, gastric and intestinal leiomyosarcoma, lung cancer, breast cancer, renal cancer, cervical cancer, ovarian cancer, prostate cancer, or head and neck tumor. In one embodiment of the present invention, the metastatic liver cancer is colon cancer metastatic liver cancer.

In one embodiment of the present invention, the combination is a combination of the compound (particularly

compound II) or the pharmaceutically acceptable salt thereof and the PD-1 antibody.

In one embodiment of the present invention, the combination is a combination of compound I or the pharmaceutically acceptable salt thereof and nofazinlimab (CS1003).

In one embodiment of the present invention, the combination is a combination of compound II or the pharmaceutically acceptable salt thereof and nofazinlimab (CS1003).

In one embodiment of the present invention, the second active ingredient is a chemotherapeutic agent, as described in the first aspect of the present invention, e.g., fluorouracil, doxorubicin, cisplatin, mitomycin, gemcitabine, capecitabine, irinotecan, sorafenib, sunitinib, lenvatinib, or erlotinib, particularly sorafenib and lenvatinib.

In one embodiment of the present invention, the combination is a combination of the compound (particularly compound II) or the pharmaceutically acceptable salt thereof and sorafenib.

In a fourth aspect of the present invention, provided is a method for preventing and/or treating a liver disease. The method comprises a step of administering to a subject in need thereof (a therapeutically effective amount) of a compound or a pharmaceutically acceptable salt thereof. The compound has the following structure:

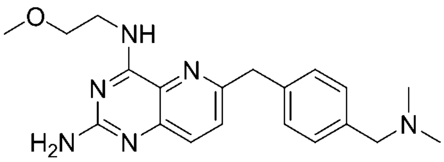

I

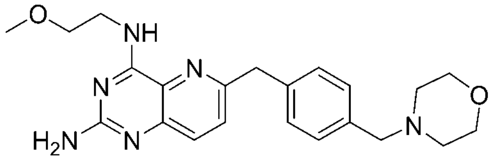

II

.

Specifically, the liver disease has the definition as described in the first aspect of the present invention. Specifically, the subject is a mammal, particularly a human.

In one embodiment of the present invention, the method is a method for preventing and/or treating liver cancer. The method comprises a step of administering to a subject in need thereof (a therapeutically effective amount) of a compound or a pharmaceutically acceptable salt thereof, and optionally, the method may further comprise other kinds of pharmaceutical formulations and/or treatment methods.

Specifically, the compound or the pharmaceutically acceptable salt thereof is administered by gastrointestinal or parenteral routes (e.g., intravenous, intramuscular, subcutaneous, intraorgan, intranasal, intradermal, instillation, intracerebral, or intrarectal administrations). In some embodiments of the present invention, the route of administration is oral administration.

Specifically, the other kinds of pharmaceutical formulations may comprise the second active ingredient as described in the first aspect of the present invention.

In one embodiment of the present invention, the method further comprises a step of administering the immune checkpoint inhibitor (as described in the first aspect of the present invention). Specifically, the method further comprises a step of administering a PD-1 antibody.

In one embodiment of the present invention, the method further comprises a step of administering the chemotherapeutic agent (as described in the first aspect of the present invention). Specifically, the method further comprises administering sorafenib or lenvatinib.

Specifically, the other kinds of treatment methods comprise, but are not limited to: hormone therapy, immunotherapy, radiotherapy, surgical treatment, and adoptive T cell therapy (e.g., TCR-T therapy and CAR-T therapy).

In some embodiments of the present invention, the route of administration of the second active ingredient is injection.

The present invention provides use of a compound in preventing and/or treating a liver disease. Experimental results show that the compound has relatively high liver tissue targeting property, extremely low cytotoxicity to primary hepatocytes, high safety for liver, and significant curative effect on liver diseases such as viral hepatitis (e.g., hepatitis B), liver cancer (including primary liver cancer and metastatic liver cancer), and the like, and also has relatively good physicochemical property, metabolic stability, bioavailability, and safety, exhibiting excellent drug development prospects.

## BRIEF DESCRIPTION OF THE DRAWINGS

FIG. 1 illustrates drug concentration change curves of compound I, compound II, and GS-9688 in the liver and plasma of ICR mice after intragastric administration.

FIG. 2 illustrates a schematic diagram of the seeding layout in a primary hepatocyte cytotoxicity assay for the compound.

FIG. 3 illustrates the results of the primary hepatocyte cytotoxicity assay for the compound.

FIG. 4 illustrates the ELISA assay results for each dose group of compound I. For each time point, from left to right, the experimental groups are vehicle control (G1), compound I-3 mpk (G2), compound I-10 mpk (G3), and compound I-20 mpk (G4), respectively.

FIG. 5 illustrates the ELISA assay results for each dose group of compound II. For each time point, from left to right, the experimental groups are vehicle control (G1), compound II-3 mpk (G5), compound II-10 mpk (G6), and compound II-20 mpk (G7), respectively.

FIG. 6 illustrates the qpcr assay results for each dose group of compound I. For each time point, from left to right, the experimental groups are vehicle control (G1), compound I-3 mpk (G2), compound I-10 mpk (G3), and compound I-20 mpk (G4), respectively.

FIG. 7 illustrates the qpcr assay results for each dose group of compound II. For each time point, from left to right, the experimental groups are vehicle control (G1), compound II-3 mpk (G5), compound II-10 mpk (G6), and compound II-20 mpk (G7), respectively.

FIG. 8 illustrates the biochemical assay results for each dose group of compound I. For each time point, from left to right, the experimental groups are vehicle control (G1), compound I-3 mpk (G2), compound I-10 mpk (G3), and compound I-20 mpk (G4), respectively.

FIG. 9 illustrates the biochemical assay results for each dose group of compound II. For each time point, from left to right, the experimental groups are vehicle control (G1), compound II-3 mpk (G5), compound II-10 mpk (G6), and compound II-20 mpk (G7), respectively.

FIG. 10 illustrates HE staining images of each experimental group in Example 5. FIGs. A to G illustrate HE staining images of vehicle control (G1), compound I-3 mpk (G2), compound I-10 mpk (G3), compound I-20 mpk (G4), compound II-3 mpk (G5), compound II-10 mpk (G6), and compound II-20 mpk (G7) experimental groups, respectively.

FIG. 11 illustrates cytokine secretion after stimulation of PBMCs from donor 1 with compound II and GS-9688.

FIG. 12 illustrates cytokine secretion after stimulation of PBMCs from donor 2 with compound II and GS-9688.

FIG. 13 illustrates cytokine secretion after stimulation of PBMCs from donor 3 with compound II and GS-9688.

FIG. 14 illustrates the evaluation results of the antiviral activity of compound II and GS-9688-stimulated PBMC supernatant, interferon $\alpha$ (positive drug), and ETV in PHHs. FIG. a illustrates the administration process, FIGs. b to d illustrate the experimental results, and A to L correspond to the results of compound II (1000 nM), compound II (333 nM), compound II (111 nM), compound II (37 nM), GS-9688 (2500 nM), GS-9688 (833 nM), GS-9688 (278 nM), GS-9688 (92.6 nM), Peg-IFN-$\alpha$2a (100 IU/mL), ETV (0.5 nM), agonist-free PBMC culture medium supernatant control, and culture medium control, respectively.

FIG. 15 illustrates the BLI values of mice in each group in the experiment of Example 7 (H22-luc orthotopic model).

FIG. 16 illustrates the imaging results of mice in each group in the experiment of Example 7 (H22-luc orthotopic model).

FIG. 17 illustrates the BLI values of mice in each group in the experiment of Example 8 (Hep3B-luc liver orthotopic tumor model).

FIG. 18 illustrates the imaging results of mice in each group in the experiment of Example 8 (Hep3B-luc liver orthotopic tumor model).

FIG. 19 illustrates the body weight changes and BLI values of mice in each group in the experiment of Example 9 (MC38-luc liver metastasis model).

FIG. 20 illustrates the BLI values of individual mice during treatment in each group in the experiment of Example 9 (MC38-luc liver metastasis model) (different lines in each figure represent the BLI values of individual mice in the corresponding group). A: vehicle, QW × 3 (n = 6); B: compound II, 3 mpk, QW × 3 (n = 6); C: PD-1, 10 mpk, BIW × 2 (n = 6); D: compound II, 3 mpk, QW × 3 + PD-1, 10 mpk, BIW × 2 (n = 6).

FIG. 21 illustrates the weights of the final tumor and liver and tumor volume of mice in each group in the experiment of Example 9 (MC38-luc liver metastasis model). A: vehicle, QW × 3 (n = 6); B: compound II, 3 mpk, QW × 3 (n = 6); C: PD-1, 10 mpk, BIW × 2 (n = 6); D: compound II, 3 mpk, QW × 3 + PD-1, 10 mpk, BIW × 2 (n = 6).

FIG. 22 illustrates the imaging results of mice in each group in the experiment of Example 9 (MC38-luc liver metastasis model).

FIG. 23 illustrates the flow cytometry panels and statistical results of lymph nodes of mice in each group in the experiment of Example 9 (MC38-luc liver metastasis model). A: vehicle (n = 5); B: compound II, 3 mpk (n = 6); C: PD-1, 10 mpk (n = 5); D: compound II, 3 mpk + PD-1, 10 mpk (n = 6).

FIG. 24 illustrates the flow cytometry panels and statistical results of the liver and tumor of mice in each group in the experiment of Example 9 (MC38-luc liver metastasis model). A: vehicle (n = 5); B: compound II, 3 mpk (n = 6); C: PD-1, 10 mpk (n = 5); D: compound II, 3 mpk + PD-1, 10 mpk (n = 6).

FIG. 25 illustrates the body weights of the male animals.

FIG. 26 illustrates the body weights of the female animals.

FIG. 27 illustrates the rectal temperature of the male animals.

FIG. 28 illustrates the rectal temperature of the female animals.

FIG. 29 illustrates the organ weights of the male animals. 1 to 9 correspond to the experimental results of the experimental groups G1 to G9, respectively.

FIG. 30 illustrates the organ weights of the female animals. 1 to 9 correspond to the experimental results of the experimental groups G1 to G9, respectively.

DETAILED DESCRIPTION

Unless otherwise defined, all scientific and technical terms used in the present invention have the same meaning as commonly understood by those skilled in the art to which the present invention relates.

In the present invention, the terms "liver disease" and "hepatic disease" may be used interchangeably and refer to a general term for all diseases occurring in the liver, including infectious diseases, neoplastic diseases, vascular diseases, metabolic diseases, toxic diseases, autoimmune diseases, hereditary diseases, hepatolithiasis, and the like. Infectious diseases further include viral infections, bacterial infections, parasitic infections, and the like, such as viral hepatitis and hepatic echinococcosis. Tumors are classified into benign tumors and malignant tumors, such as liver cancer, hepatic hemangioma, hepatic lipoma, hepatic sarcoma, and the like.

In the present invention, the term "hepatitis" refers to a general term for hepatic inflammation caused by various factors (including bacteria, viruses, parasites, alcohol, drugs, chemicals, autoimmunity, and the like). Based on the cause, hepatitis may be classified into viral, bacterial, drug-induced, alcoholic, toxic, autoimmune, and nonalcoholic steatohepatitis, and the like. Based on the duration of disease course, hepatitis may be classified into disease hepatitis and chronic hepatitis. Based on the presence or absence of jaundice, acute hepatitis may be classified into acute icteric hepatitis and acute anicteric hepatitis. Based on the severity of disease, chronic hepatitis may be classified into mild, moderate, and severe hepatitis.

The term "viral hepatitis" refers to an infectious disease primarily characterized by hepatic lesions caused by various hepatitis viruses, and mainly includes hepatitis A, hepatitis B, hepatitis C, hepatitis D, hepatitis E, and hepatitis G.

The terms "hepatic tumor" and "liver tumor" may be used interchangeably and refer to a general term for tumorous lesions occurring in the liver, which may be classified into benign tumors and malignant tumors. Benign liver tumors mainly include hepatic hemangioma, hepatic adenoma, hepatic lipoma, non-parasitic cyst of liver, focal nodular hyperplasia of liver, and the like. Malignant liver tumors mainly include primary liver cancer, secondary liver cancer, cholangiocarcinoma (particularly intrahepatic cholangiocarcinoma), and other hepatic malignancies (mainly including hepatoblastoma and hepatic sarcoma). Primary liver cancer originates from epithelial or mesenchymal tissues of the liver and includes hepatocellular carcinoma, cholangiocellular carcinoma, and mixed carcinoma of liver. Secondary or metastatic liver cancer refers to malignant tumors originating from various organs throughout the body and metastasizing to the liver. Liver metastasis is generally common in malignant tumors of organs such as stomach, biliary tract, pancreas, colorectum, ovary, uterus, lung, and breast.

The term "pharmaceutically acceptable salt" includes both acid addition salts and base addition salts.

The terms "patient" and "subject" and the like are used interchangeably herein and refer to any animal or cell thereof, whether *in vitro* or *in situ,* treated according to the method described herein. Specifically, the aforementioned animal includes mammals, for example, rats, mice, guinea pigs, rabbits, dogs, monkeys, or humans, particularly humans.

The term "treating" refers to preventing, curing, reversing, attenuating, alleviating, minimizing, suppressing, arresting, and/or stopping one or more clinical symptoms of a disease after its onset.

The term "preventing" refers to treatment to avoid, minimize, or make difficult the onset or progression of a disease prior to its onset.

The disclosures of the various publications, patents, and published patent specifications cited herein are hereby incorporated by reference in their entireties.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skill in the art without creative work shall fall within the protection scope of the present invention.

Some abbreviations and their corresponding meanings in the examples of the present invention are as follows:

| | |
|---|---|
| FBS | Fetal bovine serum |
| PBS | Phosphate buffered saline |
| P/S | Penicillin-Streptomycin |
| QW | Once a week |
| SD | Standard deviation |
| TGI | Tumor growth inhibition |
| IG | (also described as I.G., i.g. ) Intragastric administration |

IV Tail vein administration

Example 1: Preparation of Compound I

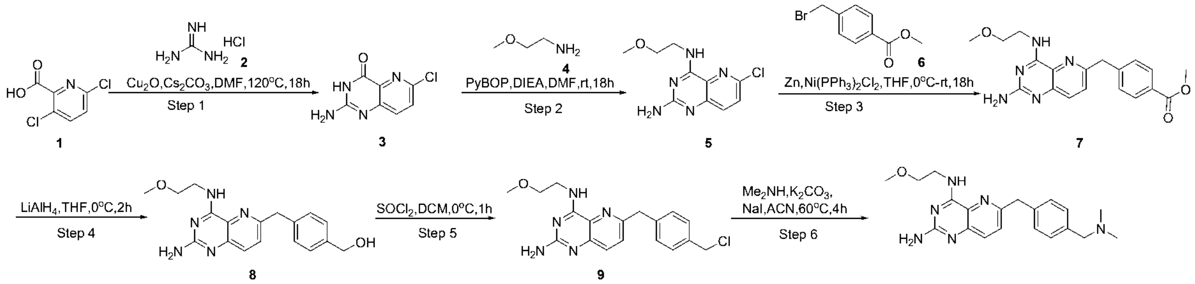


1. Synthesis of intermediate compound 3

Clopyralid (50 g, 260.4 mmol) was dissolved in N,N-dimethylformamide (800 mL), and guanidine hydrochloride (25 g, 263.2 mmol), cuprous oxide (7.4 g, 52.1 mmol), and cesium carbonate (211.6 g, 651.1 mmol) were added. The mixture was stirred at 120 °C for 18 h. The mixture was cooled to room temperature and filtered, and the filter cake was fully dissolved in methanol (1 L). The resulting solution was filtered, and the filtrate was concentrated under reduced pressure to give the crude title product as a grayish green solid 3 (50 g, crude product), which was directly used in the next step without purification. MS m/z (ESI): 197.0 [M+1].

2. Synthesis of intermediate compound 5

3 (10 g, 51.0 mmol) was dissolved in N,N-dimethylformamide (80 mL), and N,N-diisopropylethylamine (19.7 g, 153.0 mmol), 1H-benzotriazole-1-yloxytris(pyrrolidino)phosphonium hexafluorophosphate (26.5 g, 51.0 mmol), and 2-methoxyethylamine (3.8 g, 51.0 mmol) were added. The mixture was stirred at room temperature for 18 h. The reaction solution was diluted with ethyl acetate (500 mL) and washed with saturated brine (500 mL × 3). The organic phase was dried over anhydrous sodium sulfate, filtered, and concentrated under reduced pressure. The residue was purified by silica gel column chromatography (mobile phase: ethyl acetate (0.5% ammonia solution)-petroleum ether gradient: 0% to 80%) to give the title product as a yellow solid 5 (1.1 g, 8.5%). MS m/z (ESI): 254.1 [M+1].

3. Synthesis of intermediate compound 7

Methyl 4-bromomethylbenzoate (2.7 g, 11.8 mmol) was dissolved in tetrahydrofuran (80 mL), and zinc powder (2.3 g, 35.4 mmol) was added under an ice bath. The mixture was purged with nitrogen three times and stirred at 0 °C under nitrogen atmosphere for 0.5 h. Subsequently, a solution of bis(triphenylphosphine)nickel(II) chloride (780 mg, 1.2 mmol) and 5 (1.5 g, 5.9 mmol) in tetrahydrofuran (80 mL) was added at 0 °C under nitrogen atmosphere. The mixture was stirred at room temperature under nitrogen atmosphere for 18 h. The reaction solution was quenched with water (200 mL), and extracted with ethyl acetate (200 mL × 3). The organic phases were combined, dried over anhydrous sodium sulfate, filtered, and concentrated under reduced pressure. The residue was purified by silica gel column chromatography (mobile phase: methanol-dichloromethane gradient: 0% to 3%) to give the title product as a yellow solid 7 (1.1 g, 50.8%). MS m/z (ESI): 368.2 [M+1]

4. Synthesis of intermediate compound 8

7 (1.1 g, 3.0 mmol) was dissolved in tetrahydrofuran (40 mL), and a solution of lithium aluminum hydride in tetrahydrofuran (1 M) (4.5 mL, 4.5 mmol) was slowly added at 0 °C under nitrogen atmosphere. The mixture was stirred at 0 °C under nitrogen atmosphere for 2 h. The reaction solution was added dropwise to an ice-cold aqueous ammonium chloride solution (200 mL) to quench the reaction, and the mixture was extracted with ethyl acetate (200 mL × 3). The organic phases were combined, dried over anhydrous sodium sulfate, filtered, and concentrated under reduced pressure. The residue was purified by silica gel column chromatography (mobile phase: methanol-dichloromethane gradient: 0% to 4%) to give the title product as a yellow oil 8 (0.8 g, 78.7%). MS m/z (ESI): 340.1 [M+1].

5. Synthesis of intermediate compound 9

8 (0.6 g, 1.8 mmol) was dissolved in dichloromethane (20 mL), and thionyl chloride (1.1 g, 9 mmol) was slowly added at 0 °C. The mixture was stirred at 0 °C for 1 h. The reaction solution was directly concentrated under reduced pressure to give the crude title product as a brown solid 9 (0.6 g, crude product), which was directly used in the next step without purification. MS m/z (ESI): 358.2 [M+1].

6. Synthesis of compound I

9 (0.6 g, 1.7 mmol) was dissolved in acetonitrile (10 mL), and a solution of dimethylamine in tetrahydrofuran (2 M) (1 mL, 2.0 mmol), potassium carbonate (704 mg, 5.1 mmol), and sodium iodide (51 mg, 0.34 mmol) were added. The mixture was stirred at 60 °C for 4 h. The reaction solution was cooled to room temperature, quenched with water (100 mL), and extracted with ethyl acetate (100 mL × 3). The organic phases were combined, dried over anhydrous sodium sulfate, filtered, and concentrated under reduced pressure. The residue was purified by C18 silica gel column chromatography (mobile phase: acetonitrile-water (0.05% hydrochloric acid); gradient: 5% to 60%) to give the title product as a white solid I (260 mg, 41.8%). MS m/z (ESI): 367.2 [M+1]. $^1$H NMR (400 MHz, DMSO) δ 12.97 (s, 1H), 11.21 (s, 1H), 9.34 (t, *J* = 6.0 Hz, 1H), 8.48 (s, 1H), 7.87 (d, *J* = 8.6 Hz, 1H), 7.72 (d, *J* = 8.6 Hz, 1H), 7.55 (d, *J* = 8.0 Hz, 2H), 7.43 (d, *J* = 8.1 Hz, 2H), 4.84 (s, 2H), 4.41 - 4.07 (m, 4H), 3.77 (q, *J*= *5.8* Hz, 2H), 3.62 (t, *J*= 5.8 Hz, 2H), 3.30 (s, 3H), 2.63 (d, *J*= 4.8 Hz, 6H).

Example 2: Preparation of Compound II

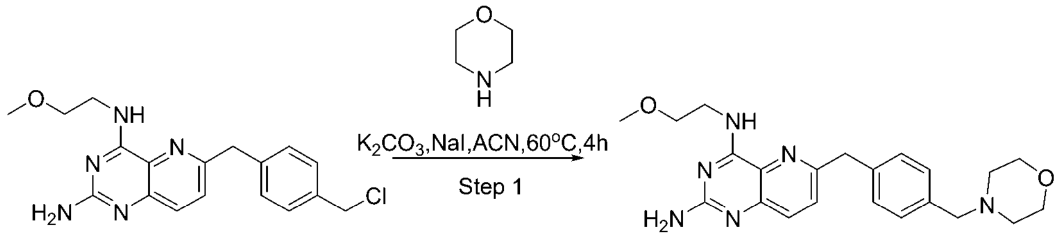


9

9 (430 mg, 1.2 mmol) (prepared in Example 1) was dissolved in acetonitrile (10 mL), and morpholine (170 mg, 2.0 mmol), potassium carbonate (500 mg, 3.6 mmol), and sodium iodide (36 mg, 0.24 mmol) were added. The mixture was stirred at 60 °C for 4 h. The reaction solution was cooled to room temperature, quenched with water (100 mL), and extracted with ethyl acetate (100 mL × 3). The organic phases were combined, dried over anhydrous sodium sulfate, filtered, and concentrated under reduced pressure. The residue was purified by C18 silica gel column chromatography (mobile phase: acetonitrile-water (0.05% trifluoroacetic acid); gradient: 5% to 60%) to give the title product as a white solid II (200 mg, 40.8%). MS m/z (ESI): 409.2 [M+1]. $^1$H NMR (400 MHz, $CD_3OD$) δ 7.73 (ddd, *J* = 8.5, 3.2, 1.5 Hz, 1H), 7.64 (dd, *J* = 8.6, 2.7 Hz, 1H), 7.45 (s, 4H), 4.30 (d, *J*= 3.7 Hz, 4H), 3.98 (d, *J* = 1.2 Hz, 2H), 3.86 (t, *J* = 5.5 Hz, 2H), 3.76 - 3.59 (m, 4H), 3.42 - 3.35 (m, 3H), 3.36 - 3.29 (m, 2H), 3.24 - 3.08 (m, 2H).

Example 3: Study on Liver Tissue Distribution of Compounds

GS-9688 was selected as the reference drug for the liver tissue distribution study. GS-9688 is an orally administered selective TLR8 agonist that is currently in Phase II clinical trials.

After single intragastric administration (10 mg/kg) of compound I, compound II, and GS-9688 (reference drug) to ICR mice, the liver and plasma were collected. The specific regimen was as follows:

Before administration, the state of the formulation for administration was inspected, and the homogeneity of the formulation was ensured by vortexing, stirring, or shaking. The theoretical dose for each mouse was calculated according to the following formula. The actual dose for each ICR mouse and the collection time for plasma and tissue samples were required to be recorded in detail in corresponding tables.

$$\text{Theoretical administration volume (mL)} = \left(\frac{\text{Dose}\ \left(\text{mg}\cdot\text{kg}^{-1}\right)}{\text{Concentration of test solution}\ \left(\text{mg}\cdot(\text{mL})^{-1}\right)}\right) \times \text{Animal body weight (kg)}$$

**Plasma sample collection:** At each designated time point, about 100 μL of blood was collected from the orbit, and the collected whole blood was placed in an EDTA-K2 anticoagulation tube, inverted several times to mix thoroughly, stored on wet ice, and centrifuged (at 1500 g to 1600 g) for 10 min within 30 min to separate the plasma. The resulting plasma sample was stored at -40 °C to -20 °C for biological sample analysis.

**The specific procedures for liver tissue collection** were **as follows:** Liver tissues were collected immediately after whole blood collection. The liver tissues were collected, washed with ice-cold normal saline, dehydrated, weighed, and homogenized in 20% methanol-water at a ratio of 1:5 (w/v). The collection time of tissues and the weight of homogenized tissues were required to be recorded in detail in corresponding tables.

The plasma and various tissue homogenates were stored in a refrigerator at -40 °C to -20 °C for sample analysis. After the experiment was completed, the mice were euthanized by carbon dioxide asphyxiation or cervical dislocation. After the analysis was completed, the samples were returned to the biological sample room, and transferred to an environment at -40 °C to -20 °C for storage according to SOP-SM-001.

**Sample analysis:** The tissue samples were analyzed by Agilent high performance liquid chromatography (HPLC, column: Waters XBridge C18 3.5 μm 2.1 × 50 mm; detector: Agilent DAD; mobile phases: phase A: H2O (0.1% formic acid), phase B: ACN (0.1% formic acid)), and the concentrations of compound I, compound II, and GS-9688 (reference drug) in the liver and plasma at each time point were determined. The corresponding pharmacokinetic parameters of plasma and liver tissues were calculated using a non-compartmental model in Phoenix WinNonlin 8.0 or later versions, and the area under curve AUC0-t (AUC0-t, the area under the concentration-time curve at 24 h post-dose) was calculated. The liver/plasma drug distribution ratio ($R_{L/P}$) was then calculated.

The liver/plasma distribution ratios of the compounds are shown in Tables 1 to 3, respectively, and the concentrations of the compounds in liver and plasma at each time point are shown in FIG. 1.

Table 1: Tissue distribution parameters of compound I (10 mg/kg) in ICR mice

| Sampling time | 0.25h | 1 h | 4h | 24h |
|---|---|---|---|---|
| Mean drug concentration (plasma) (ng/mL) | 45.4 | 25.9 | 10.7 | 1.49 |
| Mean drug concentration (liver) (ng/g) | 3416 | 2340 | 263 | 35.6 |
| Drug concentration ratio (liver/plasma) | 75.2 | 90.3 | 24.6 | 23.9 |
| $AUC_{0-t}$ (ng.h/mL) (plasma) | 206 | | | |
| $AUC_{0-t}$ (ng.h/mL) (liver) | 9480 | | | |
| $AUC_{0-t}$ ratio (liver/plasma) | 46 | | | |
| $AUC_{0-t}$, area under concentration-time curve at 24 h post-dose | | | | |

Table 2: Tissue distribution parameters of compound II (10 mg/kg) in ICR mice

| Sampling time | 0.25h | 1 h | 4h | 24h |
|---|---|---|---|---|
| Mean drug concentration (plasma) (ng/mL) | 50.8 | 65.6 | 27.9 | 4.12 |
| Mean drug concentration (liver) (ng/g) | 30980 | 8780 | 1990 | 327 |
| Drug concentration ratio (liver/plasma) | 610 | 134 | 71.3 | 79.4 |
| $AUC_{0-t}$ (ng.h/mL) (plasma) | 510 | | | |
| $AUC_{0-t}$ (ng.h/mL) (liver) | 58108 | | | |
| $AUC_{0-t}$ ratio (liver/plasma) | 114 | | | |
| $AUC_{0-t}$, area under concentration-time curve at 24 h post-dose | | | | |

Table 3: Tissue distribution parameters of GS-9688 (10 mg/kg) in ICR mice

| Sampling time | 0.25h | 1 h | 4h | 24h |
|---|---|---|---|---|
| Mean drug concentration (plasma) (ng/mL) | 364 | 70.9 | 13.8 | BLOQ |
| Mean drug concentration (liver) (ng/g) | 907 | 224 | 207 | BLOQ |
| Drug concentration ratio (liver/plasma) | 2.49 | 3.16 | 15 | NA |
| $AUC_{0-t}$ (ng.h/mL) (plasma) | 336 | | | |

(continued)

| Sampling time | 0.25h | 1 h | 4h | 24h |
|---|---|---|---|---|
| $AUC_{0-t}$ (ng.h/mL) (liver) | 1184 | | | |
| $AUC_{0-t}$ ratio (liver/plasma) | 3.5 | | | |

$AUC_{0-t}$, area under concentration-time curve at 24 h post-dose; BLOQ, below the minimum limit of detection The experimental results showed that the liver/plasma drug distribution ratio of compound I was 46-fold, the liver/plasma drug distribution ratio of compound II was 114-fold, and the liver/plasma drug distribution ratio of GS-9688 was 3.5-fold. The liver/plasma drug distribution ratio of compound I and compound II was significantly higher than that of GS-9688, and particularly, the liver/plasma drug distribution ratio of compound II was as high as 114-fold. These results indicate that compound I and compound II have relatively high liver tissue targeting properties, exhibiting a great development prospect in the treatment of liver diseases.

Example 4: Primary Hepatocyte Cytotoxicity Assay for Compound

1. Procedures

1.1. Cell seeding

(1) On day -1, primary human hepatocytes (PHHs) were thawed and seeded in a collagen-coated 96-well plate at 40000 cells/well, with a sample loading volume of 90 μL/well.
(2) The cells were cultured in an incubator at 37 °C with 5% CO2 for 4 h, and subsequently the medium was changed to the hepatocyte maintenance medium.

1.2. Compound treatment

(1) On day 0, the test compound and STS were diluted to appropriate concentrations, and the resulting solution was added at 10 μL/well. The seeding layout is illustrated in FIG. 2. The final concentrations of the test compound were 50 μM, 35 μM, 25 μM, 12.5 μM, 6.25 μM, 3.125 μM, 1.56 μM, 0.78 μM, and 0.39 μM, with 2-fold dilution to obtain 9 doses. The concentrations of the control compound *Staurosporine* (STS) were 10 μM, 3.3 μM, 1.1 μM, 0.37 μM, 0.123 μM, 0.041 μM, 0.0137 μM, 0.0045 μM, and 0.0015 μM, with 3-fold dilution to obtain 9 doses. The concentration of DMSO was 0.5%.
(2) The cells were cultured in an incubator at 37 °C with 5% CO2 for 3 days.

1.3. Proliferation assay

1.3.1. Confluence detection was performed on days 0 to 3:

(1) The cells in the 96-well plate were imaged using an IncuCyte instrument every day, and the images were analyzed using the instrument-integrated software to obtain the confluence data for the cells in each well.
(2) GraphPad Prism 8 software was used to plot graphs.

1.3.2. CTG assay was performed on day 3:

(1) A CTG assay reagent was added at 100 μL/well, and the plate was shaken to mix well (in the dark).
(2) The cells were incubated at room temperature for 30 min (in the dark).
(3) The chemiluminescence values were read using Envision.
(4) GraphPad Prism 8 software was used to plot graphs.

1.4. Data analysis

The IC50 was calculated using GraphPad Prism 8 software, and the IC50 (median inhibitory concentration) of the compounds was determined using the following nonlinear fitting formula:

$$Y = \text{Bottom} + (\text{Top} - \text{Bottom})/(1 + 10\hat{}((\text{LogIC50} - X) \times \text{HillSlope})),$$

Y: inhibition rate, X: log value of the concentration of the compound;

Inhibition (%) = 100 - (reading of compound well - low reading control well reading)/(high reading control well reading - low reading control well reading) × 100;

High reading control wells: cells plus DMSO; low reading control wells: no cell medium.

2. Experimental results

The experimental results are shown in FIG. 3. The killing activity of the positive control compound against human primary hepatocytes was 464.5 nanomolar, the killing activity of the test compound against human primary hepatocytes was 26.5 micromolar, and the test compound had weak direct killing activity against the primary hepatocytes with no apparent cytotoxicity.

Example 5: Evaluation of Liver Safety of Compounds

C57BL/6J wild-type female mice (6 weeks old) were randomly grouped. After administration at different doses, whole blood and liver tissues of the mice were separately collected at 1 h, 2 h, and 4 h for detection of a plurality of parameters to evaluate the effect of the drugs on the liver.

1. Procedures

1.1. Administration

C57BL/6J wild-type female mice (6 weeks old) were randomly grouped for administration.
Liver tissues and blood were collected at 1 h, 2 h, and 4 h after single dosing.
The experimental animals and grouping are shown in the table below.

Table 4. Experimental animals

| Animal | Number/Gender | Week age | Supplier |
|---|---|---|---|
| C57BL/6J mice | 110/Female | 6 weeks | Beijing Vital River Laboratory Animal Technology Co., Ltd. |

Table 5. Experimental grouping

| Experimental group | Experimental animal | Drug | Treatment | Administration dose | N | Time |
|---|---|---|---|---|---|---|
| G0 | Blank | None | None | N/A | 5 | None |
| G1 | C57BL/6J♀ | Vehicle | i.g. | 100 uL | 5x3 | 1 dose |
| G2 | C57BL/6J♀ | Compound I-3 mpk | i.g. | 100 uL | 5x3 | 1 dose |
| G3 | C57BL/6J♀ | Compound I-10 mpk | i.g. | 100 uL | 5x3 | 1 dose |
| G4 | C57BL/6J♀ | Compound I-20 mpk | i.g. | 100 uL | 5x3 | 1 dose |
| G5 | C57BL/6J♀ | Compound II-3 mpk | i.g. | 100 uL | 5x3 | 1 dose |
| G6 | C57BL/6J♀ | Compound II-10 mpk | i.g. | 100 uL | 5x3 | 1 dose |
| G7 | C57BL/6J♀ | Compound II-20 mpk | i.g. | 100 uL | 5x3 | 1 dose |

Preparation of compound stock solution: vehicle: 2% (v/v) DMCO + 5% PEG400 + 93% 0.01 N HCL

Samples were collected and processed as follows:

(A) 1/4 liver tissue-liquid nitrogen snap-freezing-total RNA extraction-reverse transcription-qPCR.
qPCR was performed to detect the mRNA levels of ISG15, ISG56, Mx1, and Oaslb in liver tissues of the test groups using the following primers:

internal control: mouse Gapdh
mISG15-q-F: GGTGTCCGTGACTAACTCCAT
mISG15-q-R: TGGAAAGGGTAAGACCGTCCT
mISG56-q-F: CTGAGATGTCACTTCACATGGAA
mISG56-q-R: GTGCATCCCCAATGGGTTCT
mMx1-q-F: GACCATAGGGGTCTTGACCAA
mMxl-q-R: AGACTTGCTCTTTCTGAAAAGCC
mOaslb-q-F: GGGCCTCTAAAGGGGTCAAG
mOaslb-q-R: TCAAACTTCACTCCACAACGTC.

(B) 1/4 liver tissue-homogenization by grinding-ELISA assay for later use;

(1) The liver tissues were weighed, and washed with ice-cold normal saline, and the connective tissues were removed;
(2) 5 to 8 volumes of PBS (supplemented with a protease inhibitor) were added to thoroughly homogenize the tissues, and the mixture was centrifuged at 2000 rpm for 15 min. The supernatant was collected and stored (-20 °C);
(3) IFN-α, IP-10, IL-6, and TNF-α were detected using an ELISA kit.

(C) Remaining liver tissue-HE staining and pathological evaluation.
(D) 1/2 blood (whole blood)-liver function parameter detection: AST (aspartate aminotransferase), ALT (alanine aminotransferase/glutamate pyruvate transaminase), ALP (alkaline phosphatase), ALB (albumin), and GGT (γ-glutamyl transferase).
(E) 1/2 blood-serum preparation-reserve for ELISA.

1.2. Data analysis

The experimental data were analyzed using Graphpad Prism 8.
The data were tested using 2way ANOVA.

2. Experimental results

2.1. Cytokine assay results

The ELISA assay results for each dose group of compounds I and II are shown in FIGs. 4 and 5, respectively. The results showed that the trend of change in the expression level of cytokines among different dose groups of the same drug over time was different, as detailed below:
For the compound I-3 mpk group, the expression levels of IL-6, TNF-α, and IFN-α decreased from 1 h to 4 h, and the expression level of IP-10 increased first, then decreased, and then increased from 1 h to 4 h;
For the compound I-10 mpk group, the expression levels of IL-6, TNF-α, and IFN-α increased from 1 h to 4 h, and the expression level of IP-10 peaked at 2 h and decreased at 4 h;
For the compound I-20 mpk group, the change trends of IL-6, TNF-α, IFN-α, and IP-10 were similar, and the expression levels were similar to those of the control group at 1 h, peaked at 2 h, and decreased at 4 h;
For the compound II-3/10/20 mpk/groups, the expression levels of IL-6, TNF-α, IFN-α, and IP-10 showed similar change trends, all peaked at 2 h, and decreased at 4 h. The pharmacokinetics characteristics of cytokine secretion were significant and were consistent with the absorption and metabolism of the drugs. Compound II significantly induced IP-10 (CXCL-10) secretion, and IP-10 is commonly used as one of the biomarkers for clinical evaluation of drug effect in TLR agonist clinical studies.

2.2. Assay results for interferon-related genes by real-time fluorescence quantitative PCR

The qpcr assay results for each dose group of compounds I and II are shown in FIGs. 6 and 7, respectively. The results showed that the relative expression levels of ISG15, ISG56, Mx1, and oas1b in the compound I group increased over time, and peaked at 4 h. The relative expression levels of ISG15, ISG56, Mx1, and oas1b in the compound II group increased over time, and peaked at 4 h, which were statistically significant. At a dose of 3 mpk (pharmacodynamic dose), both compound I and compound II significantly induced an increase in the mRNA expression level of interferon-related genes.

2.3. Real-time fluorescence quantitative PCR assay results

The biochemical assay results for each dose group of compounds I and II are shown in FIGs. 8 and 9, respectively. The results showed that, for compound **I,** ALT was increased in all groups, and the differences among the administration groups at 4 h were significant with statistical significance ($p < 0.001$), all exceeding the normal reference value range. However, according to the grading criteria for clinical adverse reactions, an ALT level exceeding three times the normal value corresponds to Grade 1 AE. The single administration of compound I did not result in ALT elevation to grade 1 AE. AST levels in all administration groups exceeded the normal reference range, with no statistically significant differences. ALP and GGT levels in all dose groups were within the normal reference value range. The ALB level of the compound I-10 mpk group at 2 h was significantly lower than that of the control group.

For compound II, ALT levels increased in all groups, and the differences among the administration groups at 2 h were significant with statistical significance ($p < 0.001$), exceeding the normal reference value range. However, according to the grading criteria for clinical adverse reactions, an ALT level exceeding three times the normal value corresponds to Grade 1 AE. The single administration of compound II did not result in ALT elevation to grade 1 AE. AST levels in all administration groups exceeded the normal reference range, with no statistically significant differences. ALP levels in all dose groups were within the normal reference value range. The ALB level of the compound II group at 2 h and 4 h was significantly lower than that of the control group. The GGT level of the compound II group at was significantly lower than that of the control group, and exhibited a dose-dependent relationship. If the $\gamma$-glutamyltransferase level was greater than 2.0 to 2.5 times the baseline value level, it is considered Grade 1 AE, and the lower $\gamma$-glutamyltransferase level was generally clinically normal.

2.4. Liver HE results

The livers of the mice in each dose group of compound I and compound II were collected at 1 h, 2 h, and 4 h post-dose, fixed, and then subjected to HE staining. The results showed no significant difference between each dose group and the blank group. The HE images are shown in FIG. 10, and the administration of compound I and compound II did not cause apparent pathological damage to the liver tissues.

Example 6. Assay for Anti-Hepatitis B Virus Activity of Compound

The anti-hepatitis B virus (HBV) activity of compound II and GS-9688 (reference drug) was assessed using primary human hepatocytes (PHHs), which is a classic *in vitro* infection model for studying hepatitis B virus infection.

Peripheral blood mononuclear cells (hPBMCs) from 3 donors were first stimulated with compound II and GS-9688 at 4 concentrations. After 24 h of stimulation, the culture supernatant was collected, and the cytokine concentrations of IFN-$\alpha$, IFN-$\gamma$, and IL-1240 were determined.

The cytokine secretion after stimulation of PBMCs derived from different donors by the compound is shown in FIGs. 11 to 13. In different donors, compound II induced significantly stronger activity and magnitude of IFN-$\alpha$, IFN-$\gamma$, and IL-1240 secretion than GS-9688. In particular, compound II exhibited strong IFN-$\alpha$ stimulating activity, while GS-9688 essentially failed to activate IFN-$\alpha$, which to some extent suggests that IFN-$\alpha$ is mainly produced through TLR7 activation.

The culture supernatant from one donor (donor 1) was selected to stimulate HBV-infected primary hepatocytes (PHHs), and interferon $\alpha$ and nucleoside analog entecavir (ETV) were selected as positive controls. The experimental procedures were as follows (FIG. 14a), and the detailed procedures were as follows:
On day -1, cryopreserved PHHs were thawed and adjusted to a density of $6 \times 10^5$ cells/mL, and the PHHs were seeded into a 48-well plate at 0.22 mL/well.

On day 0, the PHHs were infected with HBV type B at 600 GE/cells.

On day 1, the culture medium was replaced with a fresh one, and reference compounds ETV and Peg-IFN-$\alpha$2a (Pegasys-Roche) were added to the cells to treat the cells. The reference compounds ETV and Peg-IFN-$\alpha$2a for the PHHs were each prepared at one concentration, with three replicate wells.

On day 3, the test compound (compound II) and GS-9688-treated PBMC supernatants (donor 1), and the

reference compounds ETV and Peg-IFN-α2a were added to the cell culture medium. The PBMC supernatants were each prepared at 4 test concentrations, diluted at a ratio of 1:4, and then added to the cells. Three replicate wells were made for each test condition. The culture medium was replaced with a fresh culture medium containing the PBMC supernatants and the reference compounds on days 5, 7, 9, and 11. On day 13, the cell supernatants were collected and the cell viability was detected using CCK-8.

The content of HBV DNA in the supernatants was determined by qPCR according to the kit instructions. HBsAg and HBeAg levels in the supernatants were determined by ELISA.

The experimental results are shown in FIG. 14. Similar to the activity in inducing cytokines, compound II significantly reduced the levels of surface antigens HBeAg (FIG. 14b) and HBsAg (FIG. 14c), and also significantly reduced the level of HBV DNA (FIG. 14d). In addition, the anti-HBV activity of compound II was significantly stronger than that of the positive reference drug GS-9688. The activity of compound II for reducing hepatitis B surface antigen was slightly stronger than that of PEG-modified interferon α (Peg-IFN-α2a) (100 IU/mL), and the activity for inhibiting HBV DNA was comparable to that of PEG-modified interferon α (Peg-IFN-α2a) (100 IU/mL). As a control, the nucleoside analog ETV did not reduce the surface antigen level, but significantly inhibited HBV DNA, with activity comparable to that of compound II and GS-9688.

In conclusion, the compound II exhibited significant anti-hepatitis B virus activity and was superior to the positive reference drug GS-9688.

Example 7. Pharmacodynamic Study of Compounds in H22-luc Syngeneic Mouse Liver Cancer Model

1. Procedures

1.1. Cell culturing

H22-luc cells were rapidly thawed in a water bath at 37 °C, and 90% 1640 + 10% FBS + 1% P/S complete medium was added. The cells were placed in an incubator at 37 °C with 5% $CO_2$ and passaged every 1 to 2 days depending on the cell density.

The cell concentration (H22-luc, 2.0E8/mL) was adjusted, and the cells were mixed well with Matrigel at a ratio of 1:1. The mixture was placed on ice for subsequent injection experiments, and was mixed thoroughly by shaking with Vortex before use.

1.2. Tumor cell inoculation

(1) The mice were weighed before surgery and anesthetized by intraperitoneal injection of avertin at 0.15 mL/10 g body weight. After anesthesia, the abdominal surgical site was shaved with a pet razor, and the mice were fixed on an operating table made of foam boards.
(2) The abdominal skin preparation site was shaved and disinfected with iodophor and 75% ethanol in sequence, and a longitudinal incision of about 1.5 to 2.0 cm was made over the largest lobe of the liver with ophthalmic scissors.
(3) The largest lobe of the liver was extruded by gently pressing the costal margins on both sides with the fingers.
(4) The cells were mixed thoroughly by shaking using Vortex and pipetted using a BD brand 1-mL syringe. The needle was inserted into the liver at a 30-degree angle relative to the liver surface. After the needle was inserted, the needle was made parallel to the tabletop and advanced in parallel for about 3 mm. 10 μL of H22-luc cell suspension was slowly injected, and a rapid whitening (white plaque) or turbid translucent vacuolation over most of the liver lobe in the injection area was observed.
(5) After injection, a cotton swab was used to apply slight pressure at the needle insertion site while the needle was slowly withdrawn. The cotton swab was removed until no bleeding was observed.
(6) The liver lobe was gently pushed back into the abdominal cavity, and the muscle layer and skin were sutured. After the suture was completed, the sutured incision and the surrounding skin were wiped with 75% ethanol.
(6) After the operation, each mouse was subcutaneously injected with levofloxacin hydrochloride at a concentration of 5 mg/mL.

The experimental animals and cell lines are shown in the table below.

Table 6. Experimental animals and cell lines

| Cell | Inoculation position | Cell type | Cell passage number |
|---|---|---|---|
| H22-luc | Liver | Murine tumor cell line | 4 |
| Animal | Number/Gender | Week age | Supplier |
| BALB/c mice | 40/Female | 7 weeks | Beijing Vital River Laboratory Animal Technology Co., Ltd. |

1.3. Drug treatment

The mice were randomly grouped, and the administration information is shown below.

Table 7. Experimental grouping

| Experimental group | Number of mice | Administration dose and mode |
|---|---|---|
| A | 5 | Vehicle, 100 $\mu$L, IG, QW $\times$ 3 |
| B | 5 | Compound I-3 mpk, 100 $\mu$L, IG, QW $\times$ 3 |
| C | 5 | Compound II-3 mpk, 100 $\mu$L, IG, QW $\times$ 3 |

Preparation of compound stock solution: vehicle: 2% (v/v) DMSO + 5% PEG400 + 93% 0.01 N HCl

1.4. Data analysis

The experimental data were analyzed using Graphpad Prism 8. The experimental data were expressed as mean $\pm$ SD.

The data were tested using 2way ANOVA.

2. Experimental results

The results of the tumor inhibition effects of compound I and compound II in the H22-luc orthotopic model are shown in FIGs. 15 and 16. In the vehicle group, the mean tumor BLI reached $7.00 \times 10^{10}$ 21 days post-dose. Compound I and compound II significantly inhibited tumor growth, with TGI values of 87.0% and 83.4%, respectively ($P < 0.0001$). One mouse in the compound I group died from ascites on day 20 after grouping. No significant body weight change in the groups was observed during treatment.

3. Experimental conclusion

Compound I and compound II exhibit significant tumor inhibition effects in the H22-luc liver orthotopic tumor model.

Example 8: Pharmacodynamic Study of Compound in Hep3B-luc Human Hepatocyte Orthotopic Xenograft Mouse Model

1. Procedures

1.1. Cell culturing

Hep3B-luc cells were rapidly thawed in a water bath at 37 °C, and 90% MEM + 10% FBS + 1% P/S complete medium was added. The cells were placed in an incubator at 37 °C with 5% $CO_2$ and passaged every 1 to 2 days depending on the cell density.

The cell concentration (Hep3B-luc, 1.0E8/mL) was adjusted, and the cells were mixed well with Matrigel at a ratio of 1:1. The mixture was placed on ice for subsequent injection experiments, and was mixed thoroughly by shaking with Vortex before use.

1.2. Tumor cell inoculation

(1) The mice were weighed before surgery and anesthetized by intraperitoneal injection of avertin at 0.15 mL/10 g body weight. After anesthesia, the abdominal surgical site was shaved with a pet razor, and the mice were fixed on an operating table made of foam boards.
(2) The abdominal skin preparation site was shaved and disinfected with iodophor and 75% ethanol in sequence, and a longitudinal incision of about 1.5 to 2.0 cm was made over the largest lobe of the liver with ophthalmic scissors.
(3) The largest lobe of the liver was extruded by gently pressing the costal margins on both sides with the fingers.
(4) The cells were mixed thoroughly by shaking using Vortex and pipetted using a BD brand 1-mL syringe. The needle was inserted into the liver at a 30-degree angle relative to the liver surface. After the needle was inserted, the needle was made parallel to the tabletop and advanced in parallel for about 3 mm. 10 μL of Hep3B-luc cell suspension was slowly injected, and a rapid whitening (white plaque) or turbid translucent vacuolation over most of the liver lobe in the injection area was observed.
(5) After injection, a cotton swab was used to apply slight pressure at the needle insertion site while the needle was slowly withdrawn. The cotton swab was removed until no bleeding was observed.
(6) The liver lobe was gently pushed back into the abdominal cavity, and the muscle layer and skin were sutured. After the suture was completed, the sutured incision and the surrounding skin were wiped with 75% ethanol.
(7) After the operation, each mouse was subcutaneously injected with levofloxacin hydrochloride at a concentration of 5 mg/mL.

The experimental animals and cell lines are shown in the table below.

Table 8. Experimental animals and cell lines

| Cell | Inoculation position | Cell type | Cell passage number |
|---|---|---|---|
| Hep3B-luc | Liver | Human tumor cell line | 4 |
| Animal | Number/Gender | Week age | Supplier |
| BALB/c nude mice | 50/Female | 7 weeks | Beijing Vital River Laboratory Animal Technology Co., Ltd. |

1.3. Drug treatment

The mice were randomly grouped, and the administration information is shown below.

Table 9. Experimental grouping

| Experimental group | Number of mice | Administration dose and mode |
|---|---|---|
| A | 5 | Vehicle, 100 μL, IG, QW × 3 |
| B | 5 | Compound II-1 mpk, 100 μL, IG, QW × 3 |
| C | 5 | Compound II-3 mpk, 100 μL, IG, QW × 3 |

Preparation of compound stock solution: vehicle: 2% (v/v) DMSO + 5% PEG400 + 93% 0.01 N HCl

1.4. Data analysis

The experimental data were analyzed using Graphpad Prism 8. The experimental data were expressed as mean ± SD.
The data were tested using 2way ANOVA.

2. Experimental results

The results of the tumor inhibition effect of compound II in the Hep3B-luc liver orthotopic tumor model are shown in FIGs. 17 and 18. Compound II at 3 mg/kg significantly inhibited tumor growth ($p < 0.05$), with a TGI value of 61.6%. No

deaths occurred and no obvious clinical symptoms were observed in the administration groups. The maximum body weight loss was 8.0% after administration of compound I at 3 mg/kg and 5.4% after administration of compound II at 3 mg/kg.

3. Experimental conclusion

Compound II-3 mpk exhibits significant tumor inhibition effects in the Hep3B-luc liver orthotopic tumor model. Example 9: Pharmacodynamic Study of Compound and Administration Thereof in Combination with PD-1 in MC38-luc Syngeneic Mouse Liver Cancer Metastasis Model

1. Procedures

1.1. Cell culturing

MC38-luc cells were rapidly thawed in a water bath at 37 °C, and 90% DMEM + 10% FBS + 1% P/S + puro complete medium was added. The cells were placed in an incubator at 37 °C with 5% $CO_2$ and passaged every 1 to 2 days depending on the cell density.

The cell concentration (MC38-luc, 2.0E7/mL) was adjusted, and the cells were placed on ice for subsequent injection experiments, and were mixed thoroughly by shaking with Vortex before use.

1.2. Tumor cell inoculation

(1) The abdomen and left flank (spleen side) of the mouse were shaved and anesthetized, and the skin on the spleen side was exposed.

a. The head of the mouse was placed into the anesthetic to prevent the mouse from being awake during the operation.
b. The mouse was elevated with paper or gauze to facilitate exposure of the spleen.
c. After positioning, shaving could be performed again, and alcohol was used for wiping.

(2) At the position of the spleen, the skin and the lower mucosa were cut open with an incision of about 1 cm. The pancreas was clamped with scissors, and the spleen was gently pulled out. The spleen/pancreas was placed on moist gauze and moistened with PBS.

a. The pancreas was gently pulled out, and the spleen could not be directly pulled out as the spleen is fragile.
b. The tissue was kept moist with PBS.

(3) The spleen was gently lifted (clamped) with tweezers, and a syringe was horizontally inserted from one end of the spleen into the spleen by about 1 cm. The MC38 cells were gently injected into the spleen.

a. $1 \times 10^6$ MC38-luc, 50 μL.
b. Before pipetting the cells, the syringe was lubricated by repeatedly drawing and ejecting.
c. The injection was performed in the middle of the spleen and the spleen should not be punctured. The test fails if leakage of fluid or massive bleeding occurs during the injection.

(4) After the injection was completed, a cotton swab was used to apply pressure at the needle insertion end of the spleen, and then the needle was slowly pulled out, followed by waiting for 5 minutes to allow the cells to fully enter the bloodstream.

a. The spleen was kept moist.

(5) During the 5-min waiting period, the blood vessels at both ends of the spleen were ligated with suture threads.

a. At the bottom of the spleen, one large blood vessel at each of the anterior end and posterior end could be observed by gently breaking open the pancreas.
b. The suture thread passed through the middle position of the spleen and the pancreas, and the anterior end and

posterior end were ligated.

c. This step could prevent massive bleeding and significantly improve the success rate.

(6) After 5 min (vessel ligation), the spleen was gently clamped with tweezers, and removed (burned off) along the spleen-pancreas junction with an "electric cauterizer", and the pancreas was inserted back into the mouse.

a. The cauterizer should not be overheated, and the spleen should be cauterized (contacted) as much as possible.

b. The spleen was completely removed by cauterizing from one vascular end to the other.

(7) Suture threads were used to suture the internal mucosa (3 to 4 stitches) first and then the external skin (3 to 4 stitches).

a. Two-layer suturing was required.

b. After suturing, an analgesic agent could be immediately administered by subcutaneous injection.

The experimental animals and cell lines are shown in the table below.

Table 10. Experimental animals and cell lines

| Cell | Inoculation position | Cell type | Cell passage number |
|---|---|---|---|
| MC38-luc | Spleen | Murine colon cancer tumor cell line | 4 |
| Animal | Number/Gender | Week age | Supplier |
| C57BL/6J mice | 40/Female | 7 weeks | Beijing Vital River Laboratory Animal Technology Co., Ltd. |

1.3. Drug treatment

The mice were randomly grouped, and the administration information is shown below.

Table 11. Experimental grouping

| Experimental group | Number of mice | Administration dose and mode |
|---|---|---|
| A | 6 | Vehicle (PBS), 100 μL, IG, QW × 3 |
| B | 6 | Compound II-3 mpk, 100 μL, IG, QW × 3 |
| C | 6 | PD-1-10mpk,100 μL,IP,BIW×2 |
| D | 6 | Compound II-3 mpk, 100 μL, IG, QW × 3 + PD-1-10 mpk, 100 μL, IP, BIW × 2 |

Preparation of compound stock solution: vehicle: 2% (v/v) DMSO + 5% PEG400 + 93% 0.01 N HCl

Preparation of PD-1 antibody stock solution: vehicle: PBS; the PD-1 antibody was CS1003 antibody (sometimes referred to as "PD-1" in this example) developed by CStone Pharmaceuticals.

1.4. Data analysis

The experimental data were analyzed using Graphpad Prism 8. The experimental data were expressed as mean ± SD.

The data were tested using 2way ANOVA.

2. Experimental results

The body weight changes and BLI values of the mice in the groups during treatment with compound II and PD-1 in the MC38-luc liver metastasis model are shown in FIG. 19. The BLI value for a single mouse of each group during treatment

is shown in FIG. 20. In the vehicle group, the mean tumor BLI reached $6.24 \times 10^9$ 17 days post-dose. The administration of compound II in combination with PD-1 significantly inhibited the growth of MC38-luc tumors ($p < 0.001$), with a TGI value of 83.4%. The administration of compound II alone had an inhibitory effect on the growth of MC38-luc tumors ($p < 0.05$), with a TGI value of 63.3%. The maximum body weight loss rate in the compound II group after administration was 3.9%. The maximum body weight loss rate in the combined treatment group with compound II and PD-1 after administration was 3.8%. The synergy ratio of the combined treatment group was 2.698, indicating that the combination of the two drugs exhibited a synergistic anti-tumor effect. Final tumor and liver weights and tumor volumes are shown in FIG. 21. Imaging results of the mice in each group are shown in FIG. 22

The flow cytometry panels and statistical results for lymph nodes are shown in FIG. 23. The flow cytometry panels and statistical results for liver and tumor are shown in FIG. 24. Compound II (alone or in combination with the PD-1 antibody) significantly induced an increase in Kuffer cells in the liver tissue, which was superior to that of the vehicle group and the PD-1 antibody group. The detection results of CD8+ T cells in peripheral lymph nodes showed that there was no significant difference among the compound II group, the PD-1 group, and the vehicle group, while memory T cells were reduced.

3. Experimental conclusion

Compound II can inhibit the tumor growth in the MC38-luc syngeneic mouse liver metastasis model, and the administration of compound II in combination with PD-1 can further significantly inhibit the tumor growth in the MC38-luc syngeneic mouse liver metastasis model.

Example 10: Investigation of Physicochemical Properties of Compounds

Test compounds: compound I, compound II, and control compound N01 (the preparation of which can be found in Chinese Patent CN108069963B).

Based on the clogP (lipid-water partition coefficient) values predicted using ChemDraw software, N01 had a clogP value of 4.7, and compound I and compound II had a clogP value of3.0. The logP value was significantly decreased by more than 1.5, indicating that compound I and compound II possessed better hydrophilicity.

The solubility of the compounds in phosphate buffer at pH 7.4 was further tested, and the results showed that the solubility of compound I and compound II was improved about 10-fold compared with that of N01(D18).

The above results indicate that the compounds of the present invention exhibit better physicochemical properties and druggability.

Table 12. Lipid-water partition coefficient and solubility of compounds

| Compound | clogP[a] | Solubility (μM) (buffer, pH = 7.4) |
|---|---|---|
| N01(D18) | 4.7 | 125 |
| Compound I | 3.0 | 1215 |
| Compound II | 3.0 | 1012 |

[a.] Predicted by ChemDraw software

Example 11: Investigation of *In Vitro* Metabolic Stability of Compounds

The metabolic stability of the compounds in *in vitro* liver microsomes was evaluated. The procedures and steps were as follows:

At 37 °C, 1 μM of the compounds was incubated in 1 mg/mL liver microsomes. The reaction system was a 0.1 M potassium phosphate buffer (pH 7.4) containing 3 mmol/L $MgCl_2$ and 1 mmol/L NADPH. At the beginning of the reaction, the small molecules and the liver microsomes were pre-incubated at 37 °C for 10 min, and subsequently a cofactor NADPH was added to initiate the metabolic reaction. The reaction was quenched at 0 min, 5 min, 10 min, 20 min, 30 min, and 60 min using three volumes of ice-cold methanol containing an internal standard (200 nM labetalol). After incubation at room temperature for 10 min, the samples were centrifuged at 4000 rpm at 4 °C for 20 min, and the supernatants were collected, diluted with chromatography-grade purified water in a 1:1 ratio, and then subjected to quantitative analysis. The quantitative analysis of the compounds was performed by LC-MS/MS in an Agilent Quadrupole Time-of-Flight instrument (Agilent 6510). After a 6-minute chromatographic separation on a C18 column (50 × 2.1 mm; 2.6 μm) under a gradient mobile phase of 0.1% formic acid in water, the peaks corresponding to specified ionic signals were detected. During metabolite identification, the mobile phase gradient was 1:1, the retention time was extended to 20 min, MS/MS analysis

was performed on metabolic components, and the prominent molecular ion peaks were analyzed. The detailed operation process was as follows:

1) The compound working solution was added to all 96-well reaction plates (T0, T5, T10, T20, T30, and T60) at 10 μL/well, except the blank wells.
2) The microsomal solution was added to all the reaction wells (T0, T5, T10, T20, T30, T60, and blank wells) at 80 μL/well.
3) The plates were pre-incubated at 37 °C for 10 min.
4) After pre-incubation, NADPH was added to each reaction plate at 10 μL/well to initiate the reaction.
5) At the set end time point, a 4 °C methanol solution (containing an internal standard: 200 nM labetalol) was added to each reaction plate at 300 μL/well to quench the reaction.
6) Each plate was sealed and shaken for 10 min.
7) The mixture was shaken well and centrifuged at 4000 rpm at 4 °C for 20 min.
8) 300 μL of the supernatant was collected, and HPLC-grade water was added at 300 μL/well. The mixture was well mixed and then subjected to LC-MS analysis.

Data analysis:

The metabolic half-life (T1/2) and the metabolic clearance rate (CLint(mic)) (μL/min/mg) were calculated using a first-order kinetic equation.

First-order kinetic formula:

$$C_t = C_0 \exp(-k_e t),$$

where $k_e$ is a constant
when $C_t$ = 1/2 C0, $T_{1/2} = Ln2/k_e = 0.693/k_e$;

$$C_{Lint}(mic) = 0.693/T_{1/2} \times 1/(\text{Microsome concentration: mg/mL})$$

The results are shown in the table below.

Table 13. Evaluation of liver microsome stability of compounds

| Compound | Species | k(l/min) | $T_{1/2}$(min) | $CL_{int,microsome}$ (μL/min/mg) |
|---|---|---|---|---|
| N01(D18) | Human | 0.136 | 5.1 | 136 |
| | Murine | 0.08 | 8.6 | 80 |
| Compound I | Human | 0.00683 | 102 | 13.7 |
| | Murine | 0.732 | 0.947 | 1465 |
| Compound II | Human | 0.0197 | 35.1 | 39.5 |
| | Murine | 0.0460 | 15.1 | 92.0 |

The assay results showed that compound I had an extremely short half-life in mouse liver microsomes, while it had a significantly prolonged half-life in human liver microsomes. The half-life of compound II was significantly prolonged in both human and mouse liver microsomes. This indicated that oxygen substitution of the butyl chain alleviated the metabolic rate, and the oxidation at the β position of the N01 alkyl chain was presumed to be a potential metabolic pathway. The above results indicate that compounds I and II may have better oral bioavailability and metabolic stability, particularly in humans.

Example 12: Investigation of Inhibitory Activity of Compounds Against Cardiac Potassium Channel (hERG)

The rapidly activated potassium channel encoded by human ether-a-go-go-related gene (hERG) is an important ion channel involved in the formation of cardiac action potential phase 3 repolarization. Drug-induced blockade of hERG

channels can lead to delayed cardiac repolarization, manifested as QT interval prolongation on the electrocardiogram, known as long QT syndrome. Drug-induced delayed ventricular repolarization may, under certain circumstances, trigger fatal arrhythmias-torsade de pointes-type ventricular tachycardia. In this study, the whole-cell patch-clamp technique (the standard method for hERG safety assessment) was employed to evaluate the inhibitory effects of the small-molecule compounds on the hERG potassium channel and to assess the risk of ventricular repolarization toxicity.

The assay results are shown in the table below. Compound N01 exhibited strong inhibitory activity against hERG, with $IC_{50}$ < 3 μM, approaching the threshold of clinical drug safety requirements. Compared with NO1, compound I and compound II exhibited significantly reduced overall lipophilicity and significantly reduced inhibitory activity against hERG. Compound II had moderate hERG inhibitory activity ($IC_{50}$ = 4.3 μM), and compound II had weak inhibition according to the general standard ($IC_{50}$ > 10 μM). The assay results showed that compounds I and II, particularly compound I, significantly improved cardiac safety. Considering that the doses of compound I and compound II were very low, according to the ICH cardiac safety assessment criterion ($C_{max}$/hERG $IC_{50}$ > 30 indicates low risk), the $C_{max}$/hERG $IC_{50}$ of compound I and compound II was greater than 30, indicating a low risk of cardiotoxicity.

Table 14. Evaluation results of hERG inhibitory activity of compounds

| Compound | hERG ($IC_{50,}$μM) |
|---|---|
| N01(D18) | 2.1 |
| Compound I | 17.2 |
| Compound II | 4.3 |
| Cisapride | 0.013 |

Example 13: Toxicity Evaluation of Compounds in Single Administration

The purpose of this study was to evaluate the tolerance of C57BL/6 mice subjected to single intragastric administration of the test compounds, compound I and compound II, at different doses, and subsequently maintained for 7 days, to observe clinical symptoms (toxic reactions) of the mice and to assess the maximum tolerated dose (MTD) of the mice.

1. Procedures

1.1. Experimental animals

C57BL/6 mice, 8 weeks old, female/male, 27, supplier: SPF (Beijing) Biotechnology Co., Ltd.

The experimental animals were housed in a SPF-grade laminar flow clean room at constant temperature and humidity in individual cages. The housing room was maintained at 22 ± 3 °C with 40% to 70% humidity and a 12-hour light/dark cycle.

1.2. Animal grouping and treatment

The animals were randomly divided into 8 groups based on the body weight of the animals. The detailed groups are shown below.

Table 15. Animal grouping

| Group | Drug | Group treatment | Number of animals (female/male) | Administration volume (10 mL/kg) | Route of administration | Frequency of administration |
|---|---|---|---|---|---|---|
| G1 | Compound I | 10 mg/kg | 3/3 | 10 | Intragastric administration | Single administration |
| G2 | | 20 mg/kg | 3/3 | 10 | Intragastric administration | Single administration |
| G3 | | 30 mg/kg | 3/3 | 10 | Intragastric administration | Single administration |
| G4 | | 40 mg/kg | 3/3 | 10 | Intragastric administration | Single administration |
| G 5 | Compound II | 10 mg/kg | 3/3 | 10 | Intragastric administration | Single administration |
| G6 | | 20 mg/kg | 3/3 | 10 | Intragastric administration | Single administration |
| G7 | | 30 mg/kg | 3/3 | 10 | Intragastric administration | Single administration |
| G8 | | 40 mg/kg | 3/3 | 10 | Intragastric administration | Single administration |
| G9 | Vehicle group | | 3/3 | 10 | Intragastric administration | Single administration |

The method for preparing the stock solutions of the compounds is shown in the table below.

Table 16. Preparation of compound stock solution

| Test compound | Preparation method | Administration volume (mL/kg) | Concentration (mg/mL) |
|---|---|---|---|
| Vehicle | 2% ethanol + 10% PEG300 + 88% 0.01 N HCl | | |
| Compound I @ 40 mpk | Solution A1: 25 mg of compound I was first added to 0.125 mL of ethanol, and the mixture was vortexed and sonicated until | 10 | 4 |
| | dissolution. Subsequently, 0.625 mL of PEG300 was added, and the mixture was vortexed and sonicated. Finally, 5.5 mL of 0.01 N HCl was added, and the mixture was vortexed and sonicated to obtain solution A1. | | |
| Compound I @ 30 mpk | Solution A2: 3.75 mL of solution A1 was added to 1.25 mL of vehicle, and the mixture was vortexed and sonicated to obtain solution A2. | 10 | 3 |
| Compound I @ 20 mpk | Solution A3: 2.4 mL of solution A2 was added to 1.2 mL of vehicle, and the mixture was vortexed and sonicated to obtain solution A3. | 10 | 2 |
| Compound I @ 10 mpk | Solution A4: 1.25 mL of solution A3 was added to 1.25 mL of vehicle, and the mixture was vortexed and sonicated to obtain solution A4. | 10 | 1 |
| Compound II @ 40 mpk | Solution B1: 25 mg of compound II was first added to 0.125 mL of ethanol, and the mixture was vortexed and sonicated until dissolution. Subsequently, 0.625 mL of PEG300 was added, and the mixture was vortexed and sonicated. Finally, 5.5 mL of 0.01 N HCl was added, and the mixture was vortexed and sonicated to obtain solution B1. | 10 | 4 |

(continued)

| Test compound | Preparation method | Administration volume (mL/kg) | Concentration (mg/mL) |
|---|---|---|---|
| Compound II @ 30 mpk | Solution B2: 3.75 mL of solution B1 was added to 1.25 mL of vehicle, and the mixture was vortexed and sonicated to obtain solution B2. | 10 | 3 |
| Compound II @ 20 mpk | Solution B3: 2.4 mL of solution B2 was added to 1.2 mL of vehicle, and the mixture was vortexed and sonicated to obtain solution B3. | 10 | 2 |
| Compound II @ 10 mpk | Solution B4: 1.25 mL of solution B3 was added to 1.25 mL of vehicle, and the mixture was vortexed and sonicated to obtain solution B4. | 10 | 1 |

1.3. Measurement parameters and method

1.3.1. Body weight

The body weight of the animals was measured and recorded daily.

1.3.2. Clinical observation

All groups were subjected to clinical observations before administration and 1 to 2 h after administration, and additional observations were performed if necessary. Deaths and mortality were checked twice daily. The observations should include, but are not limited to, the following: fur condition, activity, mental status, vomiting, diarrhea, and abnormal secretions around the eyes. All animals should be observed on the day of necropsy, and any abnormal findings should be recorded.

Animals were euthanized if the following conditions occurred:

The animal had lost a substantial amount of body weight (emaciation). The cumulative body weight loss was equal to or greater than 20% compared with the body weight on day 1.

The animal was unable to obtain sufficient food or water.

The animal showed physical weakness, impaired movement, or low body temperature.

1.3.3. Rectal temperature measurement

The rectal temperature of all animals was measured and recorded at 0 h before administration and at 2 h, 4 h, 8 h and 24 h after administration.

1.3.4. Gross necropsy and organ weighing

The experiment was terminated on day 7 after administration. A gross necropsy was performed to examine abdominal and thoracic organs for lesions, and the spleen and liver were collected for weighing.

1.4. Statistics

The experimental results were expressed as "mean ± standard deviation". The data of each group were statistically analyzed using SPSS17.0 software package. One-way ANOVA test was employed to compare data among groups so as to determine statistical differences. A value of $P < 0.05$ was considered statistically significant.

2. Experimental results

2.1. Body weight

The body weight measurement results of the experimental animals are shown in FIGs. 25 and 26. In male animals, the body weight of the animals treated with compound I and compound II decreased after the single administration, began to recover from day 3 to day 7, and the body weight of the animals in groups 1, 2, 3, 4, 6, 7, and 8 significantly decreased on day 2 compared with that of the vehicle group. In female animals, the body weight of the animals treated with compound I and compound II decreased after the single administration, began to recover from day 3 to day 7, the body weight of the animals in groups 1, 2, 3, 4, 6, 7, and 8 significantly decreased on day 3 compared with that of the

vehicle group, and the body weight of the animals in groups 2, 3, 4, and 8 significantly decreased on day 4 compared with that of the vehicle group.

2.2. Rectal temperature

The rectal temperature measurement results of the experimental animals are shown in FIGs. 27 and 28. After the animals were treated with compound I and compound II for 2 h, the rectal temperature of the animals decreased, exhibiting a significant difference compared with that of the vehicle group. The rectal temperature of the animals gradually returned to normal 4 h, 8 h and 24 h after the treatment.

2.3. Cage-side observation

During the experiment, male animal No. 46 in group 1 treated with compound I at 10 mg/kg was observed to have a mild hunched posture on day 2, and male animal No. 35 in group 6 treated with compound II at 20 mg/kg was observed to have white secretions around the eyes on day 2. No abnormalities were observed in the remaining animals.

2.4. Gross necropsy

During necropsy, an enlarged spleen was observed in male animal No. 46 in group 1 treated with compound I at 10 mg/kg and female animal No. 2 in group 4 treated with compound I at 40 mg/kg. No obvious gross necropsy abnormalities were observed in the remaining animals.

2.5. Organ weight

The weight measurement results of the spleen and liver of the animals are shown in FIGs. 29 and 30. In the female animals, the spleen weight of animals treated with compound I at 30 mg/kg was significantly increased compared with that of the vehicle group.

3. Experimental conclusion

In conclusion, under the current experimental conditions, the maximum tolerated dose of compound I and compound II for both female and male animals was 40 mg/kg.

When tested under the same experimental conditions, the maximum tolerated dose of compound N01 was 20 mg/kg, the incidence of adverse reactions with compound I and compound II was lower, and the body weight loss was milder. The experimental results indicate that compound I and compound II have better safety.

Table 17. Evaluation results of toxicity of compounds in single administration

| Compound | Maximum tolerated dose (MTD) | Adverse reactions at maximum tolerated dose/probability | Weight loss rate at maximum tolerated dose/whether recovered | With or without mouse death |
|---|---|---|---|---|
| N01(D18) | 20 mg/kg | White secretion around the eyes (1/6) | 17%/Yes | No |
| Compound I | 40 mg/kg | No | 12.5%/Yes | No |
| Compound II | 40 mg/kg | White secretion around the eyes (1/6) | 14%/Yes | No |

The above description is only for the purpose of illustrating the preferred examples of the present invention, and is not intended to limit the scope of the present invention. Any modifications, equivalent substitutions, and the like made without departing from the spirit and principle of the present invention shall fall within the protection scope of the present invention.

The foregoing examples and methods described herein may vary based on the abilities, experience, and preferences of those skilled in the art.

The certain order in which the steps of the method are listed in the present invention does not constitute any limitation on the order of the steps of the method.

## Claims

1. Use of a compound or a pharmaceutically acceptable salt thereof in preparing a medicament for preventing and/or treating a liver disease, wherein the compound has the following structure:

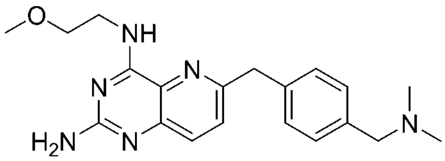 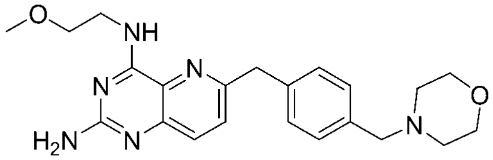

.

2. The use according to claim 1, wherein the liver disease is hepatitis, preferably selected from: viral hepatitis, alcoholic hepatitis, steatohepatitis, drug-induced hepatitis, and autoimmune hepatitis, more preferably viral hepatitis.

3. The use according to claim 2, wherein the viral hepatitis is selected from: hepatitis A, hepatitis B, hepatitis C, hepatitis D, hepatitis E, and hepatitis G, preferably hepatitis B.

4. The use according to claim 1, wherein the liver disease is a liver tumor, preferably selected from: hepatic hemangioma, hepatic adenoma, hepatic lipoma, non-parasitic cyst of liver, focal nodular hyperplasia of liver, liver cancer, bile duct cancer, hepatoblastoma, and hepatic sarcoma, preferably liver cancer.

5. The use according to claim 4, wherein the liver cancer is primary liver cancer or metastatic liver cancer; preferably, a primary tumor of the metastatic liver cancer is selected from: colorectal cancer, colon cancer, gastric cancer, pancreatic cancer, gastric and intestinal leiomyosarcoma, lung cancer, breast cancer, renal cancer, cervical cancer, ovarian cancer, prostate cancer, and head and neck tumor.

6. The use according to claim 1, wherein the liver disease is liver injury, preferably hepatic fibrosis and cirrhosis; or the liver disease is fatty liver or hepatolithiasis.

7. The use according to claim 1, wherein the medicament further comprises a second active ingredient selected from one or more of a radiotherapeutic agent, a chemotherapeutic agent, an immunotherapeutic agent, an antiangiogenic agent, a cytokine, a hormone, a polynucleotide, an antibody, an immunologically active fragment, an immune checkpoint blocking agent, a cell therapy drug, a small nucleic acid drug, an mRNA vaccine, and a tumor vaccine;

   preferably, the immune checkpoint is selected from: PD-1, PD-L1, CTLA-4, BTLA, TIM-3, LAG-3, TIGIT, LAIR1, 2B4, and CD160, preferably PD-1 or PD-L1;
   preferably, the chemotherapeutic agent is selected from: fluorouracil, doxorubicin, cisplatin, mitomycin, gemcitabine, capecitabine, irinotecan, sorafenib, sunitinib, erlotinib, and lenvatinib.

8. The use according to claim 7, wherein the second active ingredient is a PD-1 antibody, e.g., pembrolizumab, nivolumab, cemiplimab, nofazinlimab (CS1003), tislelizumab, toripalimab, sintilimab, camrelizumab, penpulimab, zimberelimab, or serplulimab; or

   the second active ingredient is a PD-L1 antibody, e.g., atezolizumab, duvalumab, durvaluamb, sugemalimab, or envafolimab; or
   the second active ingredient is a CTLA-4 antibody, e.g., tremelimumab or ipilimumab; or
   the second active ingredient is a multi-target receptor tyrosine kinase (RTK) inhibitor, e.g., sorafenib or lenvatinib.

9. The use according to any one of claims 1 to 8, wherein the medicament further comprises one or more pharmaceutically acceptable auxiliary materials.

10. The use according to claim 9, wherein a dosage form of the medicament is a tablet, a pill, a powder, a granule, a capsule, a lozenge, a syrup, a liquid, an emulsion, a suspension, an injection, a spray, an aerosol, or a powder aerosol; preferably, the medicament is in an oral dosage form.

11. The use according to any one of claims 1 to 8, wherein a route of administration of the compound or the

pharmaceutically acceptable salt thereof is oral administration.

**12.** Use of a combination of a compound or a pharmaceutically acceptable salt thereof and a second active ingredient in preparing a medicament for preventing and/or treating a liver disease, wherein the compound has the following structure:

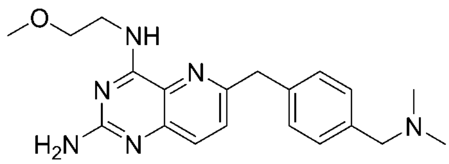

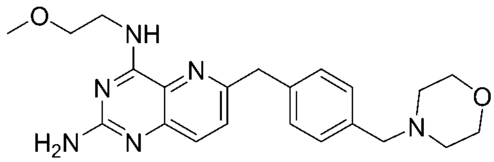

the second active ingredient is selected from one or more of a radiotherapeutic agent, a chemotherapeutic agent, an immunotherapeutic agent, an antiangiogenic agent, a cytokine, a hormone, a polynucleotide, an antibody, an immunologically active fragment, an immune checkpoint blocking agent, a cell therapy drug, a small nucleic acid drug, an mRNA vaccine, and a tumor vaccine;
preferably, the immune checkpoint is selected from: PD-1, PD-L1, CTLA-4, BTLA, TIM-3, LAG-3, TIGIT, LAIR1, 2B4, and CD160, preferably PD-1 or PD-L1;
preferably, the chemotherapeutic agent is selected from: fluorouracil, doxorubicin, cisplatin, mitomycin, gemcitabine, capecitabine, irinotecan, sorafenib, sunitinib, erlotinib, and lenvatinib.

**13.** The use according to claim 12, wherein the liver disease is a liver tumor, preferably liver cancer.

**14.** The use according to claim 12 or 13, wherein the combination is a combination of the compound or the pharmaceutically acceptable salt thereof and a PD-1 antibody;
preferably, the compound is compound II.

**15.** The use according to any one of claims 12 to 14, wherein a route of administration of the compound or the pharmaceutically acceptable salt thereof is oral administration; and/or a route of administration of the second active ingredient is injection.

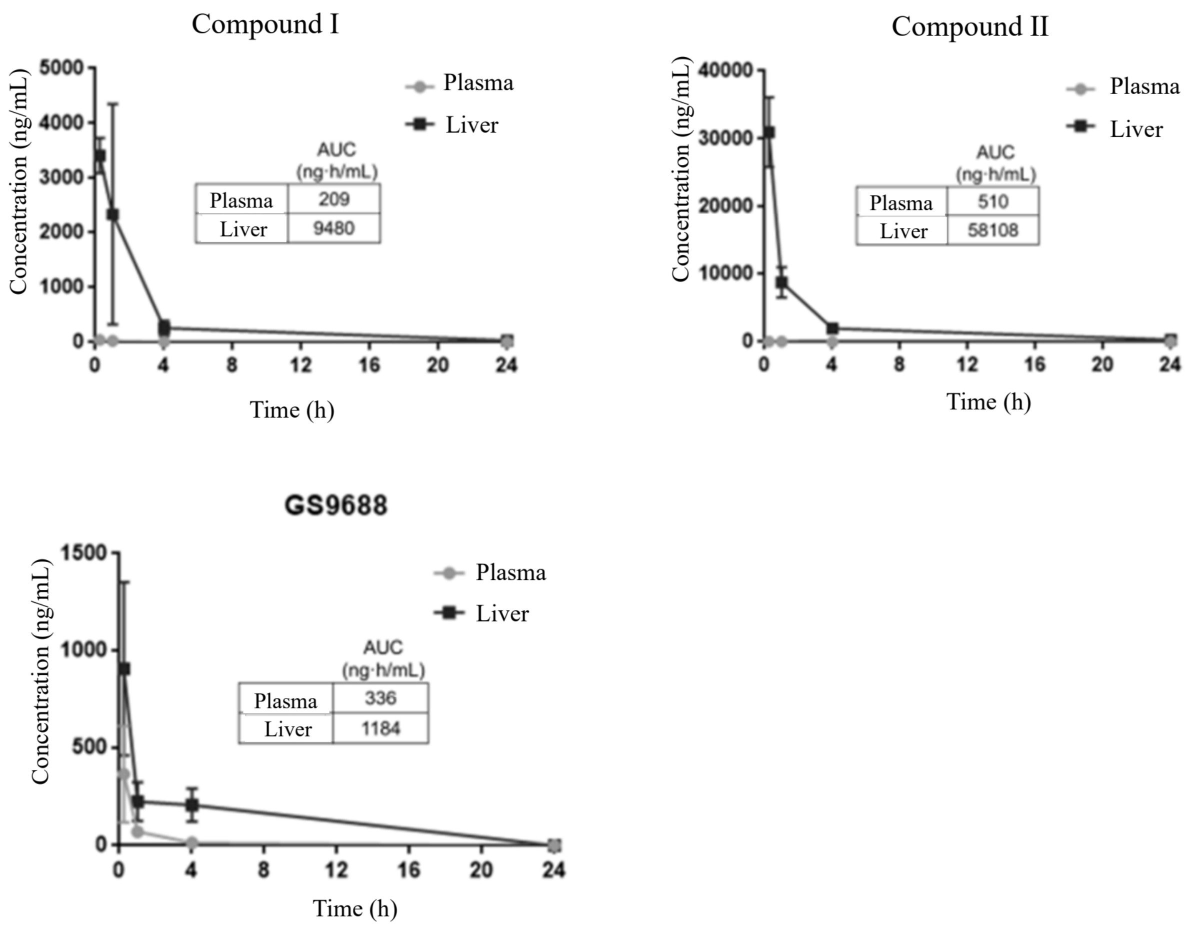


FIG. 1

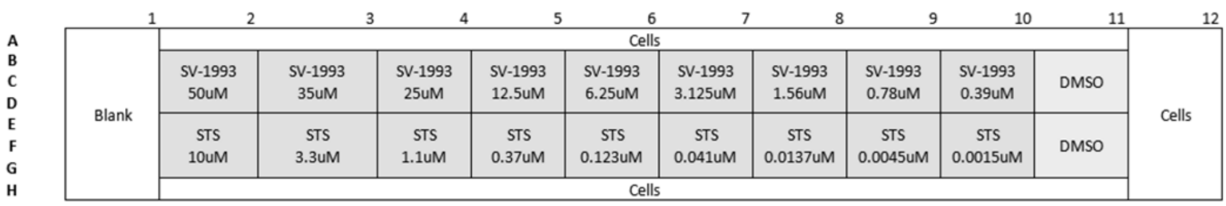


FIG. 2

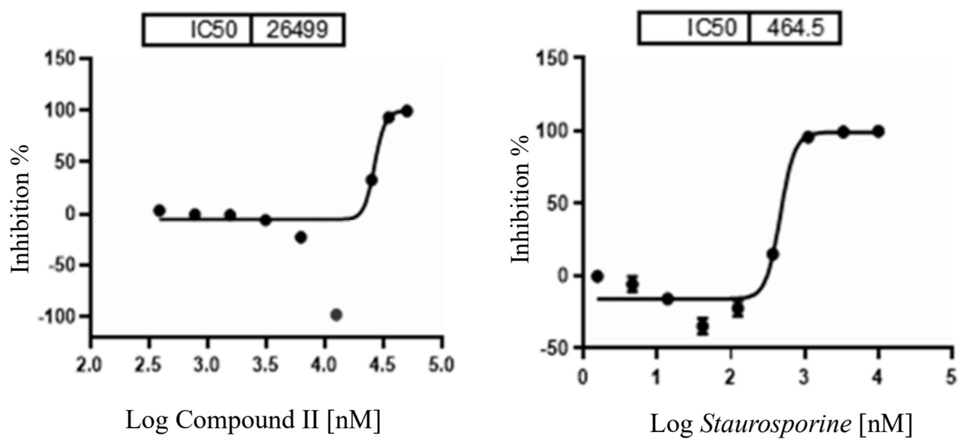


FIG. 3

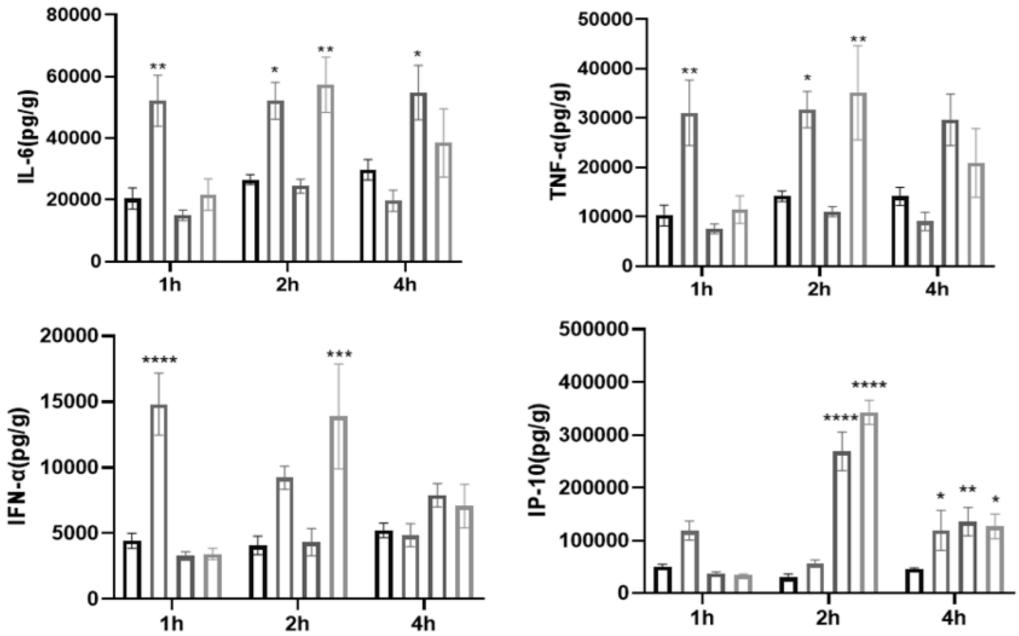
IL-6(pg/g)
TNF-α(pg/g)
IFN-α(pg/g)
IP-10(pg/g)
1h
2h
4h

FIG. 4

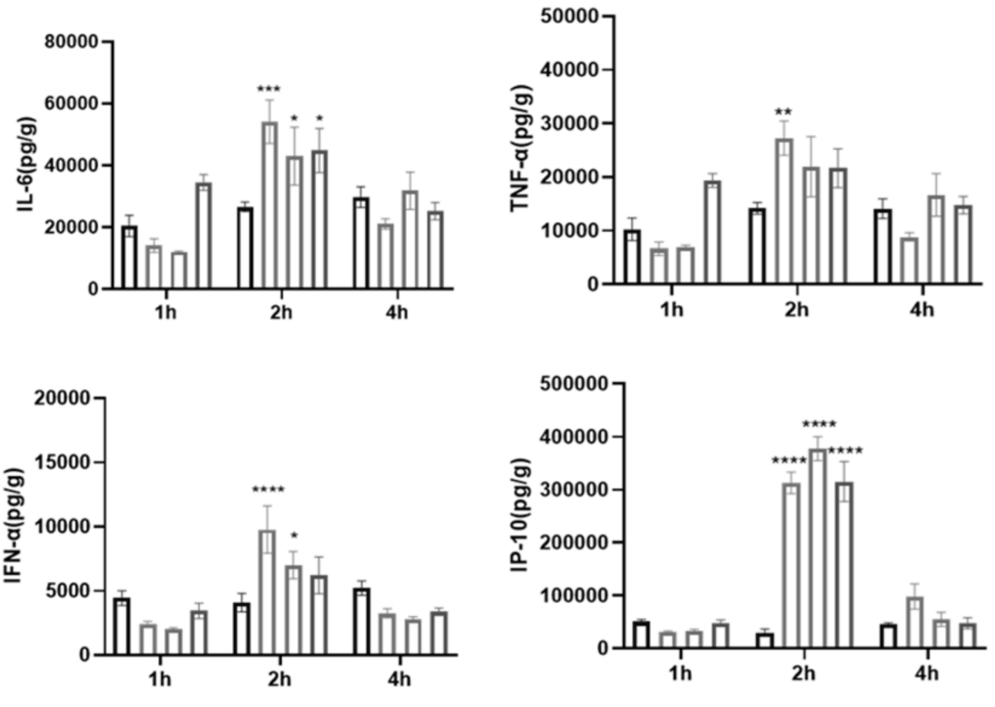
IL-6(pg/g)
TNF-α(pg/g)
IFN-α(pg/g)
IP-10(pg/g)
1h
2h
4h

FIG. 5

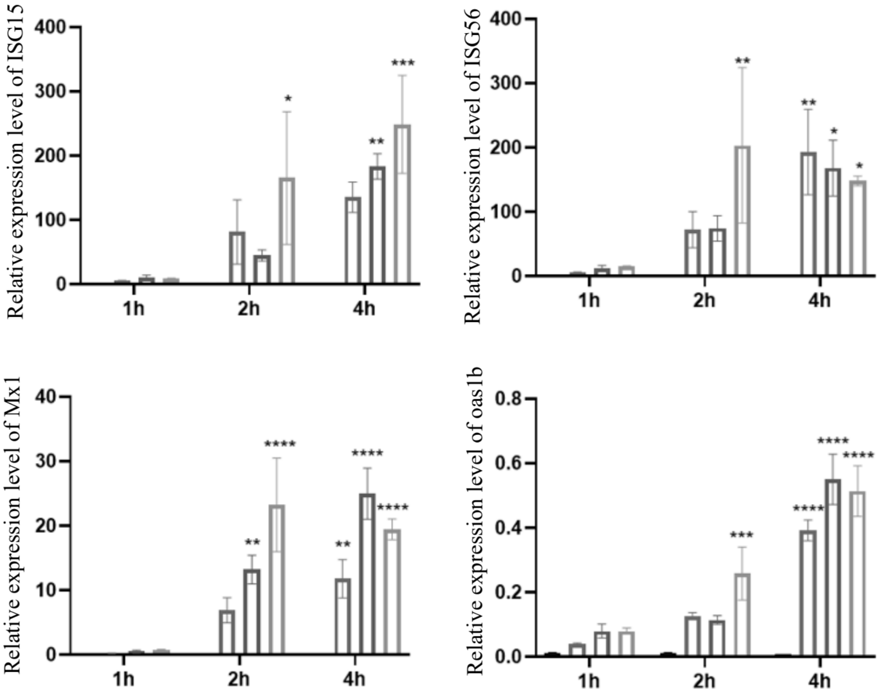
Relative expression level of ISG15
Relative expression level of ISG56
Relative expression level of Mx1
Relative expression level of oas1b
1h
2h
4h

FIG. 6

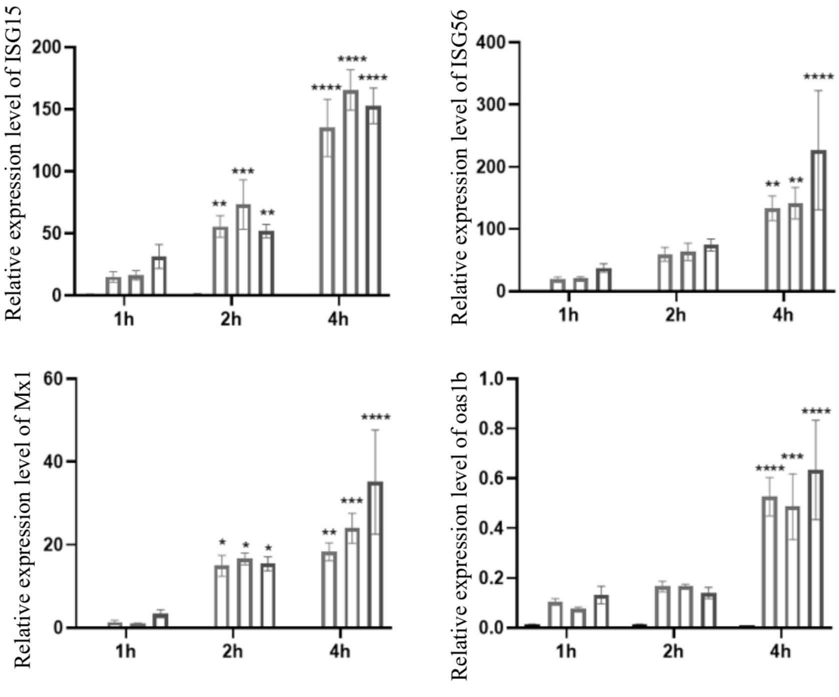
Relative expression level of ISG15
Relative expression level of ISG56
Relative expression level of Mx1
Relative expression level of oas1b
1h
2h
4h

FIG. 7

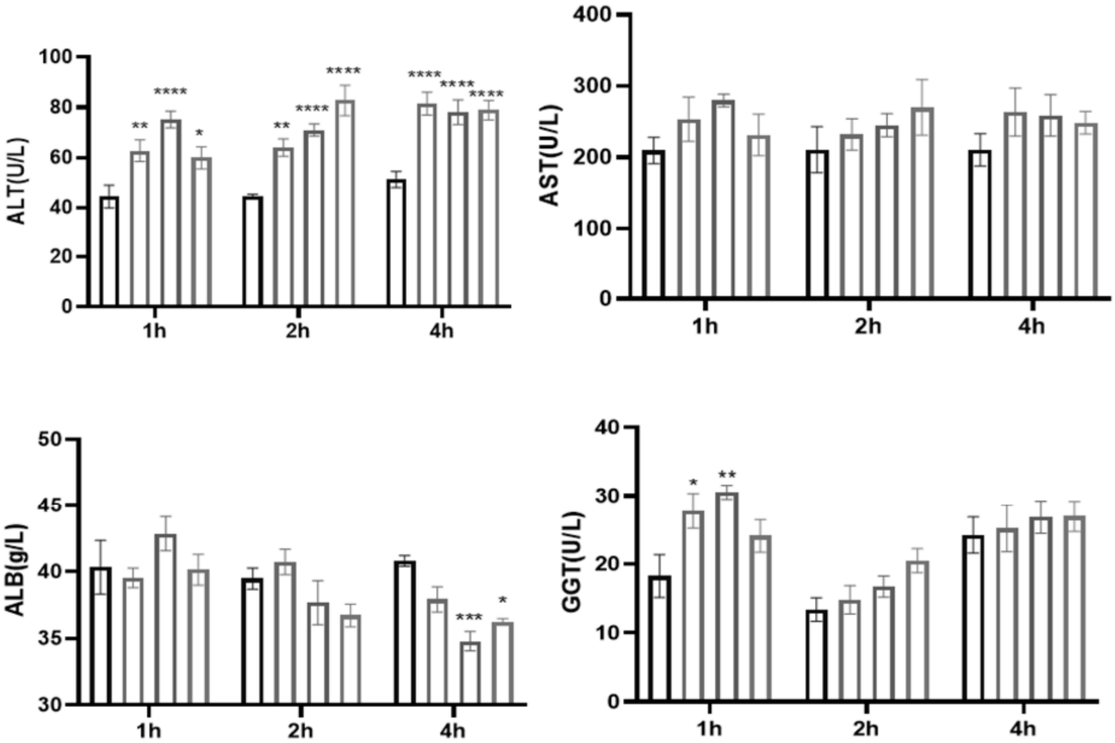
100
80
60
40
20
0
ALT(U/L)
**
****
*
**
****
****
****
****
****
1h
2h
4h
400
300
200
100
0
AST(U/L)
1h
2h
4h
50
45
40
35
30
ALB(g/L)
***
*
1h
2h
4h
40
30
20
10
0
GGT(U/L)
*
**
1h
2h
4h

FIG. 8

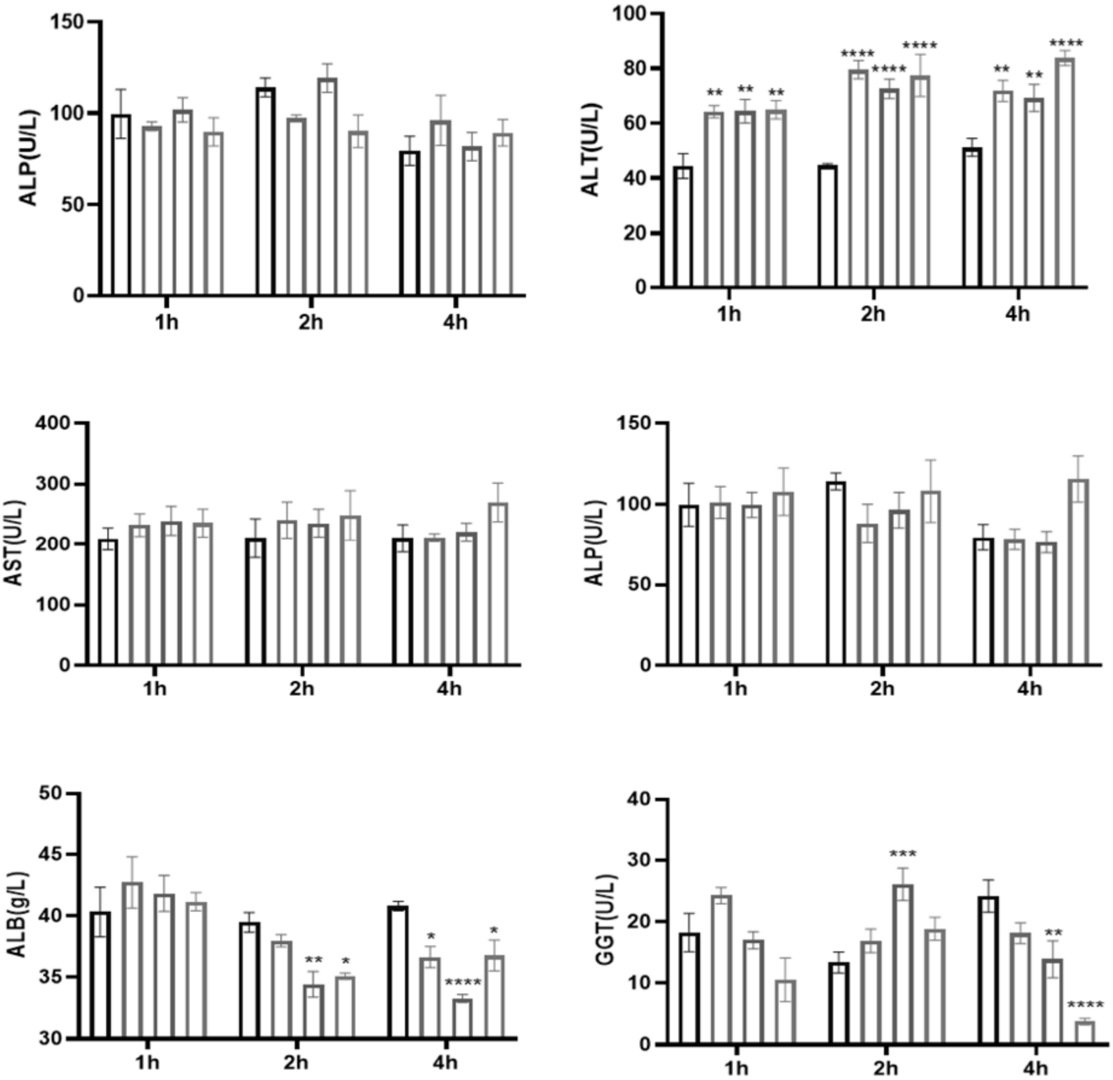
150
100
50
0
ALP(U/L)
1h
2h
4h
100
80
60
40
20
0
ALT(U/L)
**
**
**
****
****
****
**
**
****
1h
2h
4h
400
300
200
100
0
AST(U/L)
1h
2h
4h
150
100
50
0
ALP(U/L)
1h
2h
4h
50
45
40
35
30
ALB(g/L)
**
*
*
****
*
1h
2h
4h
40
30
20
10
0
GGT(U/L)
***
**
****
1h
2h
4h

FIG. 9

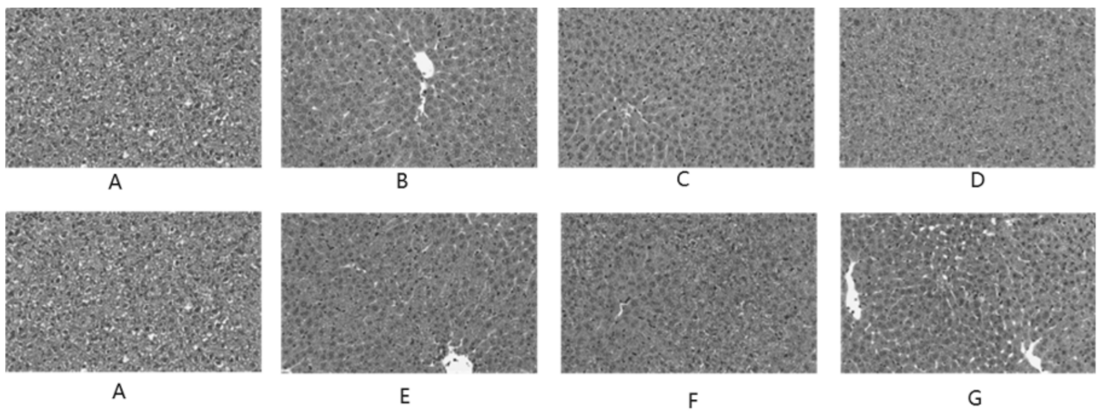
A
B
C
D
A
E
F
G

FIG. 10

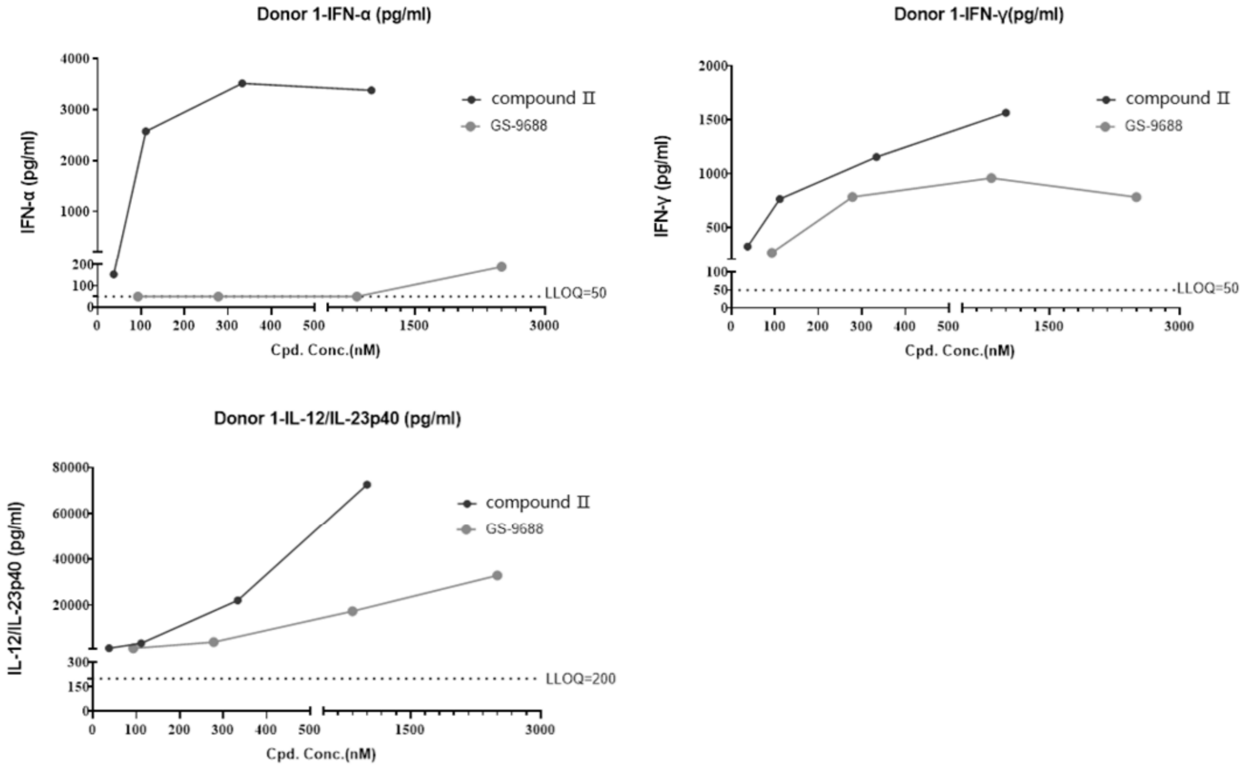
Donor 1-IFN-α (pg/ml)
IFN-α (pg/ml)
Cpd. Conc.(nM)
compound II
GS-9688
LLOQ=50
Donor 1-IFN-γ(pg/ml)
IFN-γ (pg/ml)
Cpd. Conc.(nM)
compound II
GS-9688
LLOQ=50
Donor 1-IL-12/IL-23p40 (pg/ml)
IL-12/IL-23p40 (pg/ml)
Cpd. Conc.(nM)
compound II
GS-9688
LLOQ=200

FIG. 11

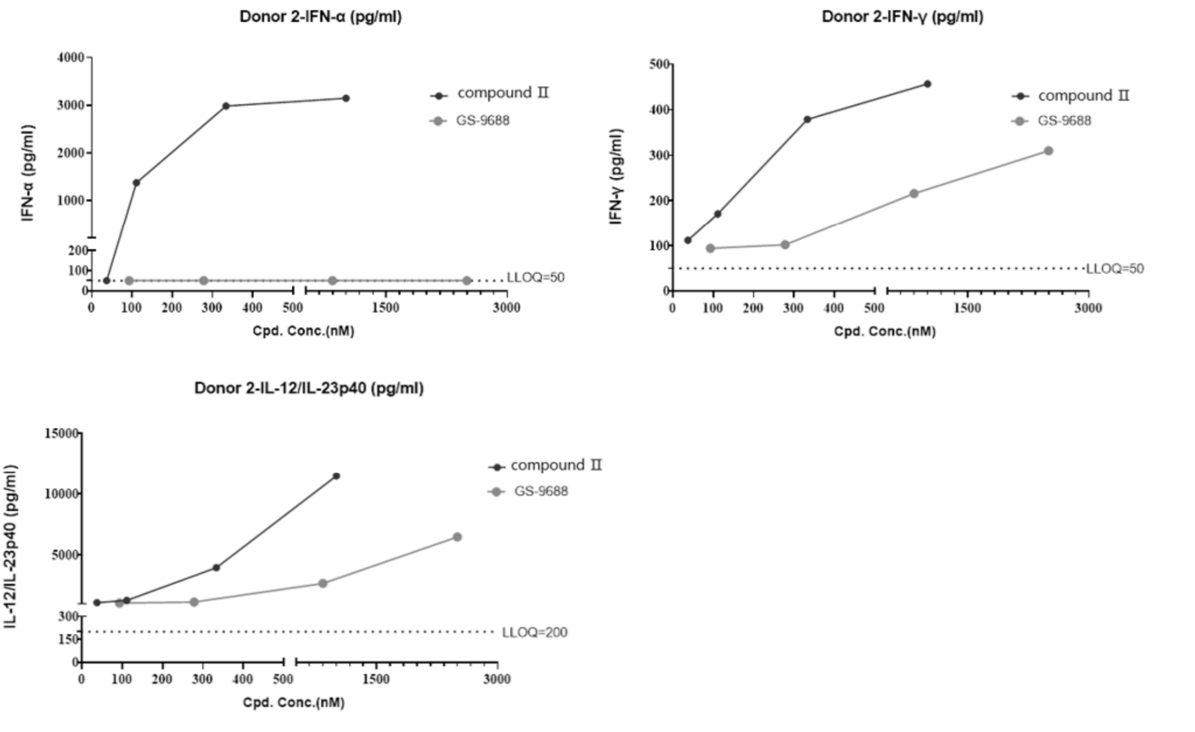
Donor 2-IFN-α (pg/ml)
IFN-α (pg/ml)
Cpd. Conc.(nM)
compound II
GS-9688
LLOQ=50
Donor 2-IFN-γ (pg/ml)
IFN-γ (pg/ml)
Cpd. Conc.(nM)
compound II
GS-9688
LLOQ=50
Donor 2-IL-12/IL-23p40 (pg/ml)
IL-12/IL-23p40 (pg/ml)
Cpd. Conc.(nM)
compound II
GS-9688
LLOQ=200

FIG. 12

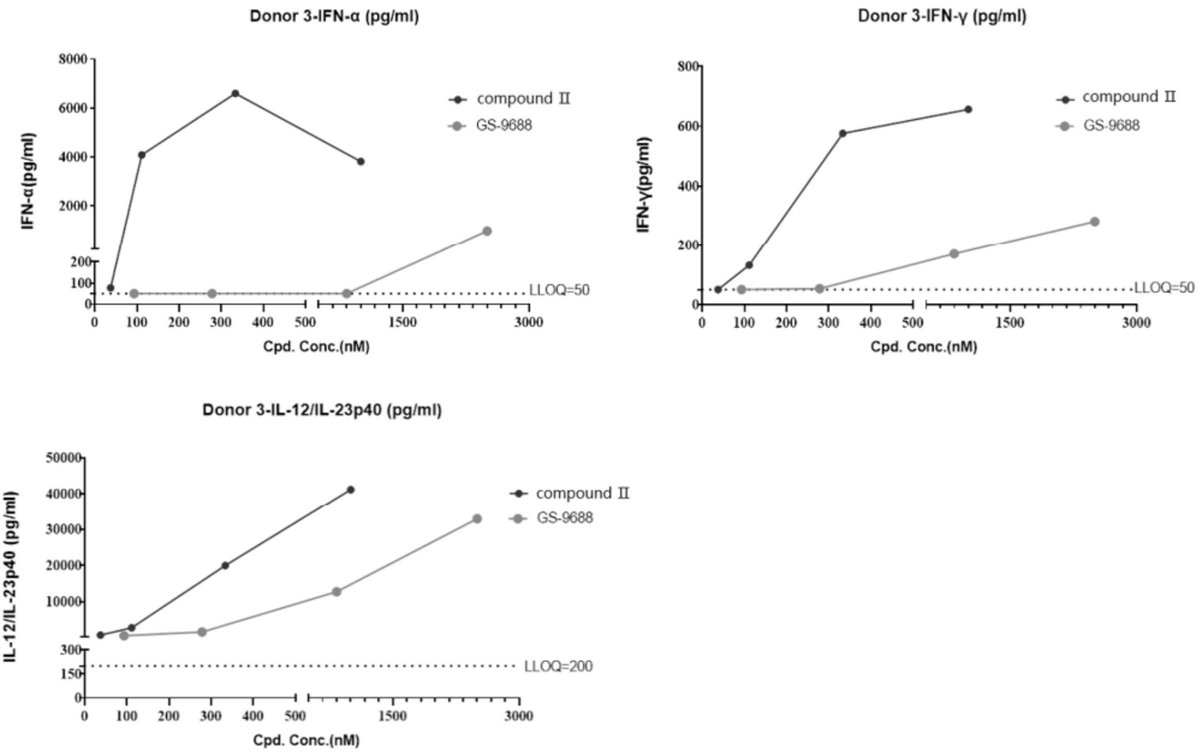
Donor 3-IFN-α (pg/ml)
IFN-α(pg/ml)
8000
6000
4000
2000
200
100
0
compound Ⅱ
GS-9688
LLOQ=50
0
100
200
300
400
500
1500
3000
Cpd. Conc.(nM)
Donor 3-IFN-γ (pg/ml)
IFN-γ(pg/ml)
800
600
400
200
0
compound Ⅱ
GS-9688
LLOQ=50
Cpd. Conc.(nM)
Donor 3-IL-12/IL-23p40 (pg/ml)
IL-12/IL-23p40 (pg/ml)
50000
40000
30000
20000
10000
300
150
0
compound Ⅱ
GS-9688
LLOQ=200
Cpd. Conc.(nM)

FIG. 13

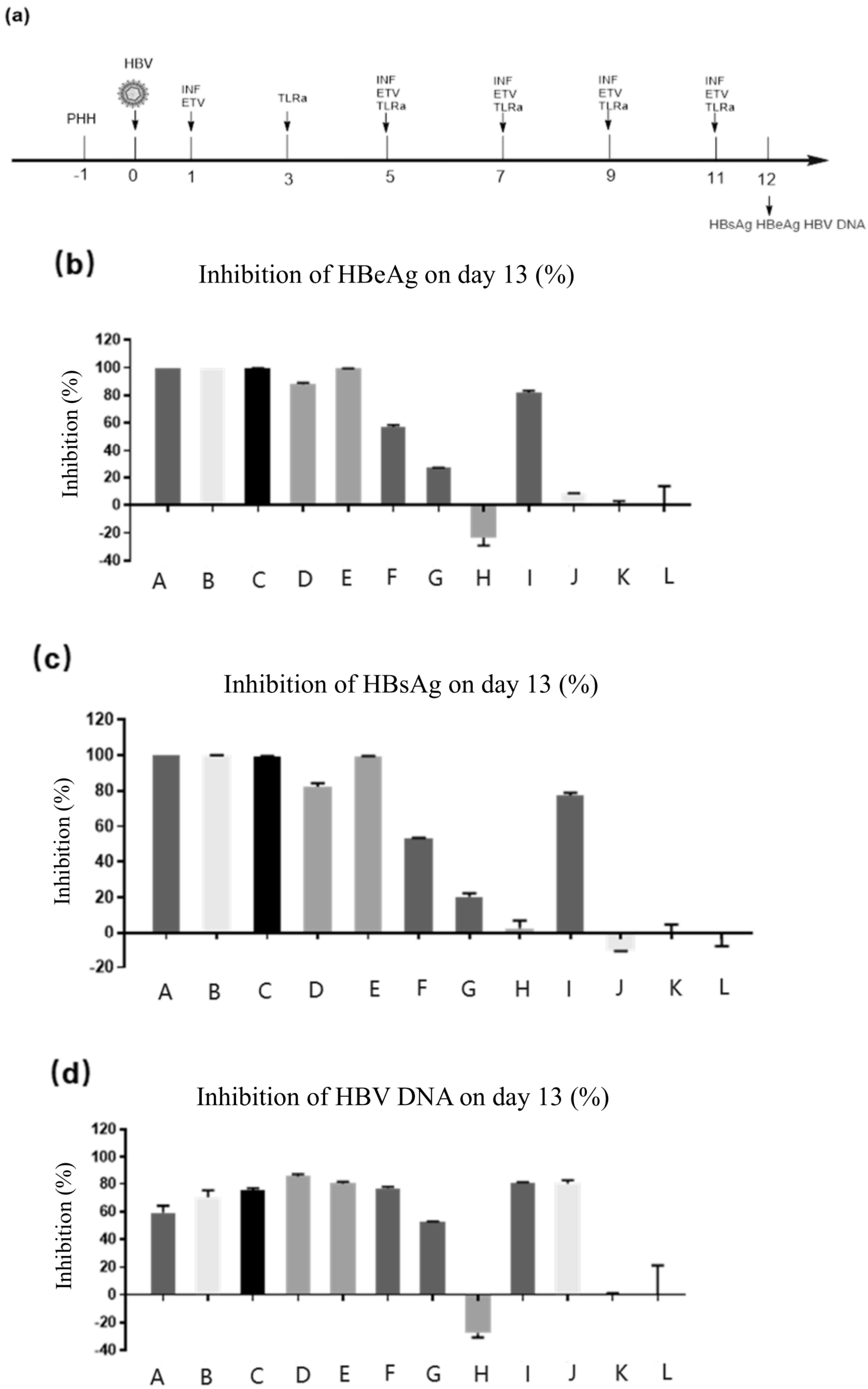
(a)
HBV
PHH
INF
ETV
TLRa
-1 0 1 3 5 7 9 11 12
HBsAg HBeAg HBV DNA
(b)
Inhibition of HBeAg on day 13 (%)
Inhibition (%)
120 100 80 60 40 20 0 -20 -40
A B C D E F G H I J K L
(c)
Inhibition of HBsAg on day 13 (%)
Inhibition (%)
120 100 80 60 40 20 0 -20
A B C D E F G H I J K L
(d)
Inhibition of HBV DNA on day 13 (%)
Inhibition (%)
120 100 80 60 40 20 0 -20 -40
A B C D E F G H I J K L

FIG. 14

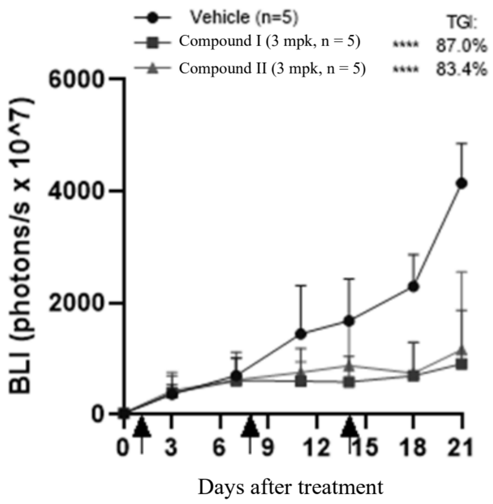
Vehicle (n=5)
Compound I (3 mpk, n = 5) **** 87.0%
Compound II (3 mpk, n = 5) **** 83.4%
TGI:
BLI (photons/s x 10^7)
6000
4000
2000
0
0 3 6 9 12 15 18 21
Days after treatment

FIG. 15

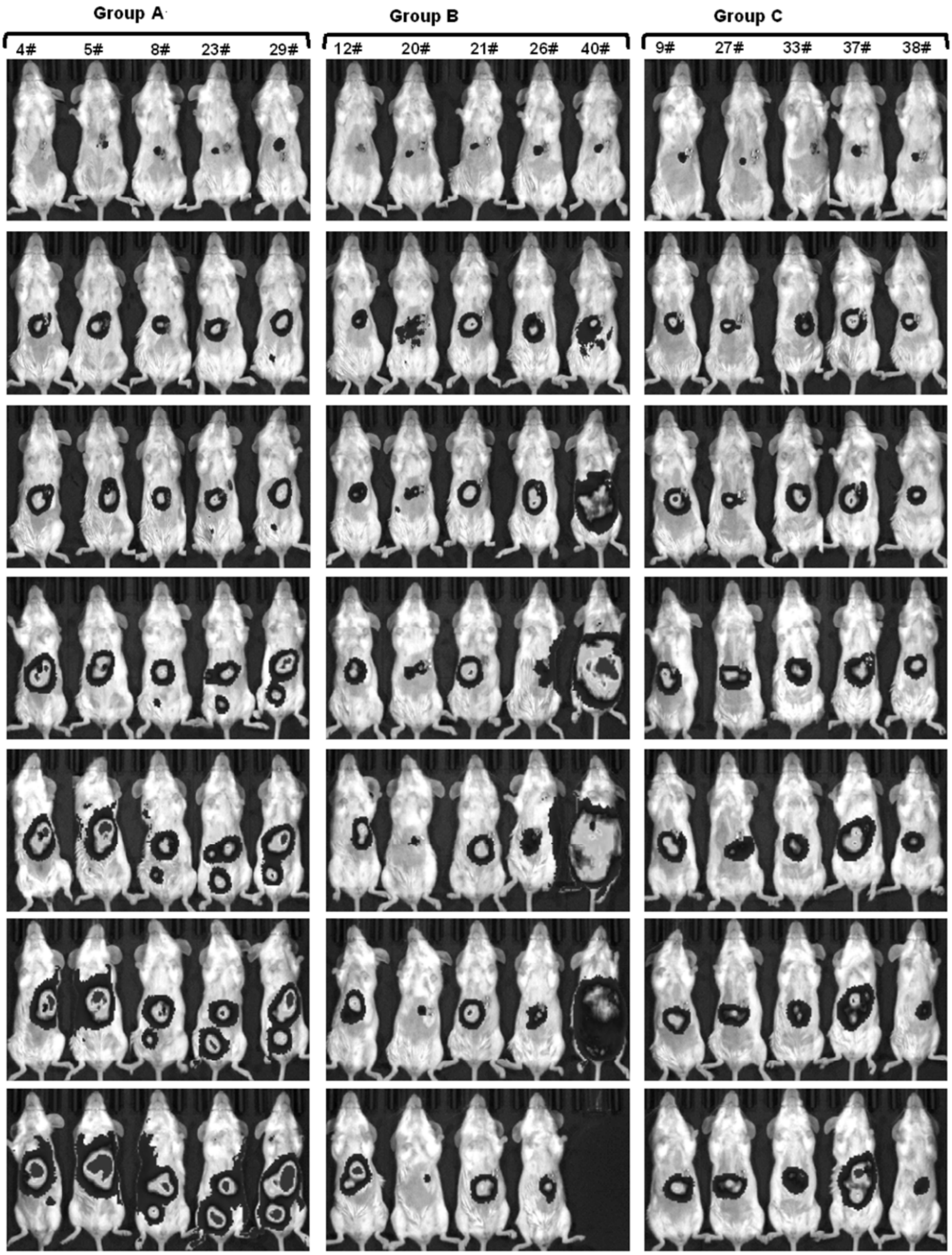


FIG. 16

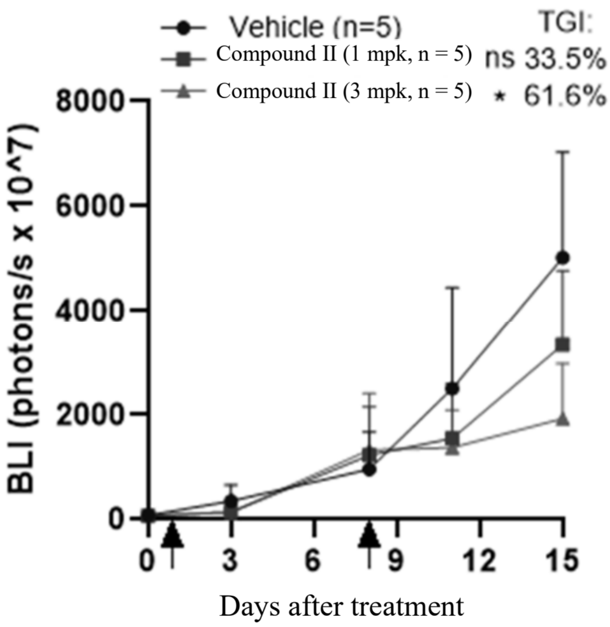
Vehicle (n=5)
TGI:
Compound II (1 mpk, n = 5)
ns 33.5%
Compound II (3 mpk, n = 5)
* 61.6%
BLI (photons/s x 10^7)
8000
6000
4000
2000
0
0
3
6
9
12
15
Days after treatment

FIG. 17

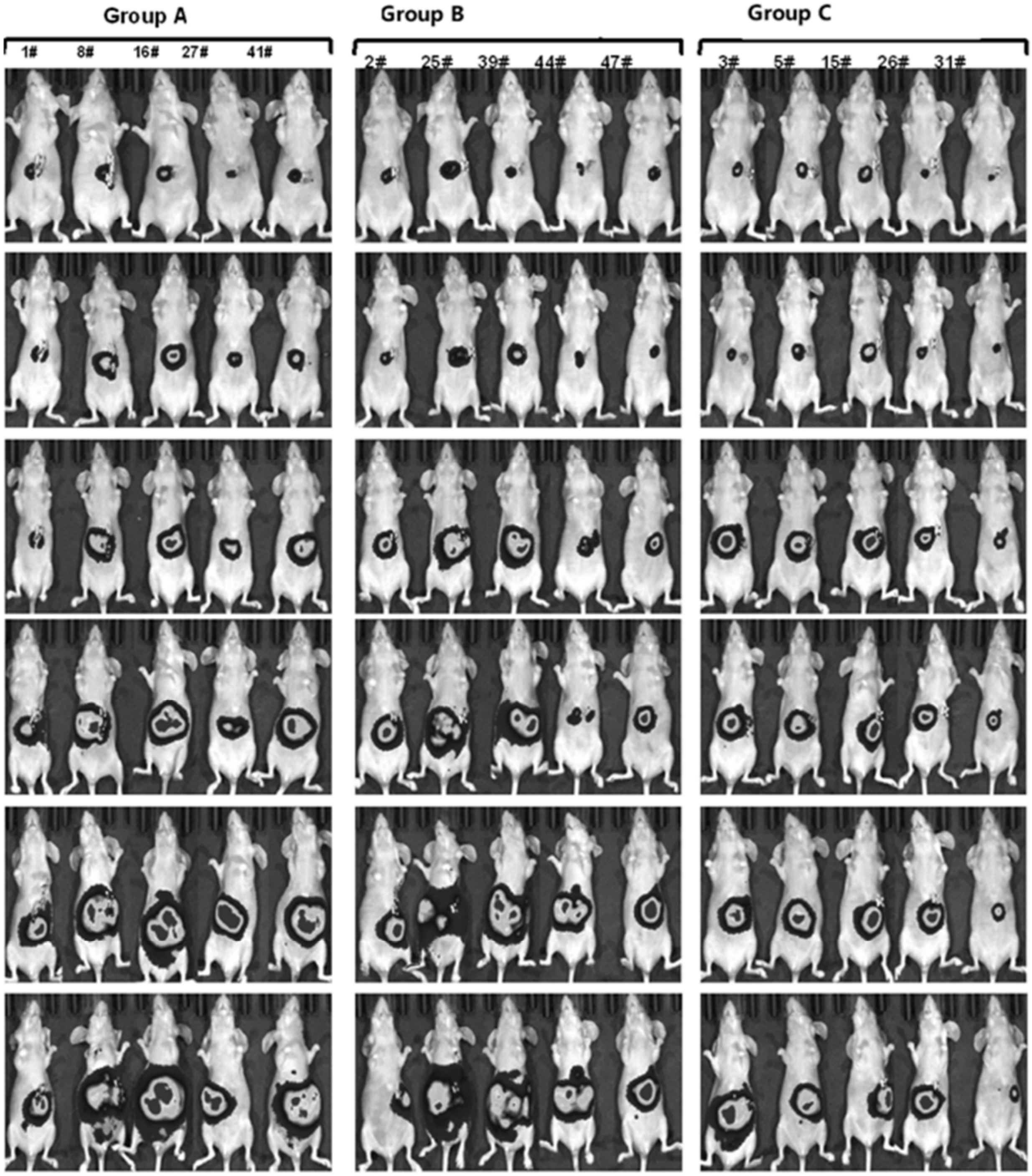


FIG. 18

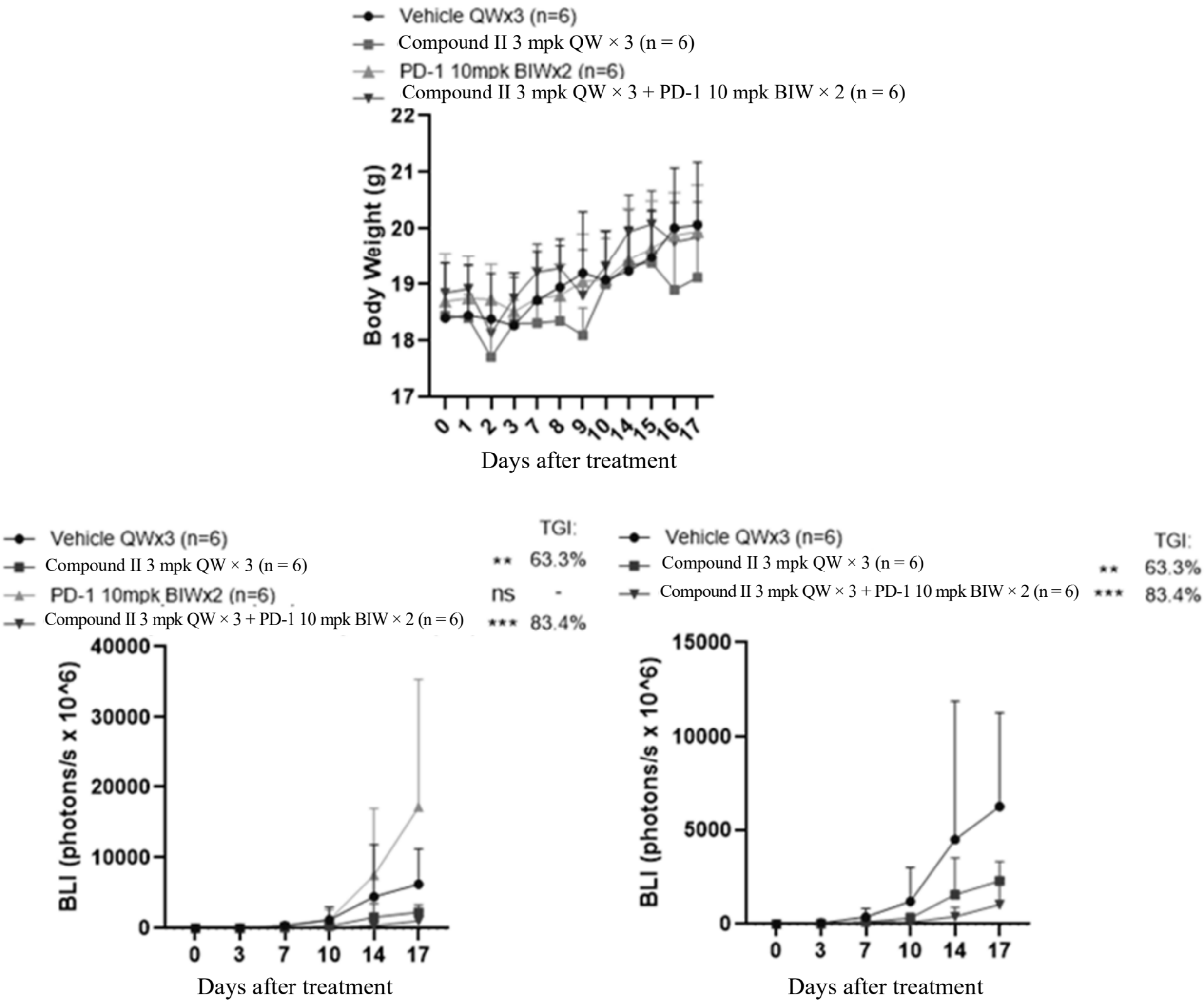
Vehicle QWx3 (n=6)
Compound II 3 mpk QW × 3 (n = 6)
PD-1 10mpk BIWx2 (n=6)
Compound II 3 mpk QW × 3 + PD-1 10 mpk BIW × 2 (n = 6)
Body Weight (g)
Days after treatment
TGI:
** 63.3%
ns -
*** 83.4%
BLI (photons/s x 10^6)
Days after treatment
Vehicle QWx3 (n=6)
Compound II 3 mpk QW × 3 (n = 6)
Compound II 3 mpk QW × 3 + PD-1 10 mpk BIW × 2 (n = 6)
TGI:
** 63.3%
*** 83.4%
BLI (photons/s x 10^6)
Days after treatment

FIG. 19

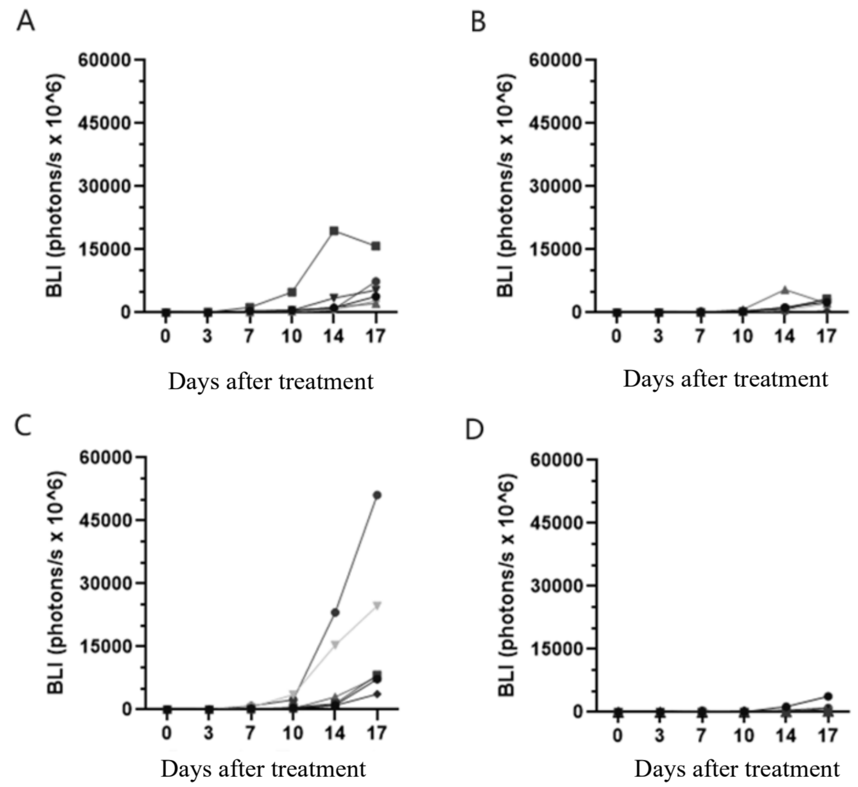
A
B
C
D
BLI (photons/s x 10^6)
60000
45000
30000
15000
0
0 3 7 10 14 17
Days after treatment

FIG. 20

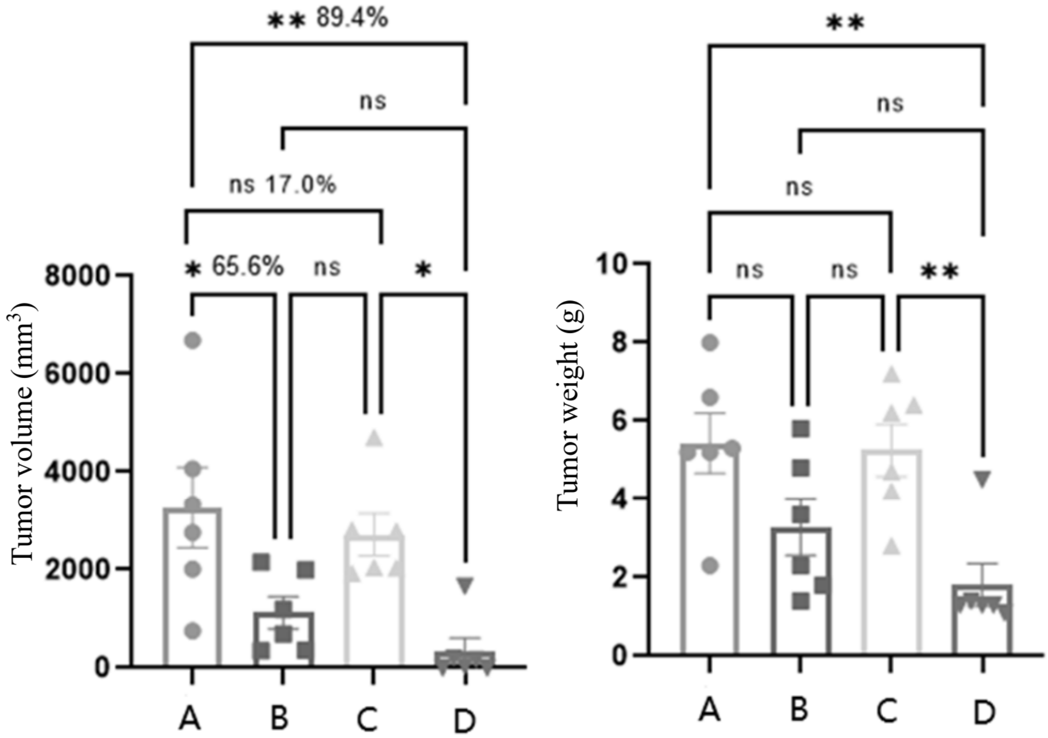
** 89.4%
ns
ns 17.0%
* 65.6%
ns
*
Tumor volume (mm3)
8000
6000
4000
2000
0
A B C D
**
ns
ns
ns
ns
**
Tumor weight (g)
10
8
6
4
2
0
A B C D

FIG. 21

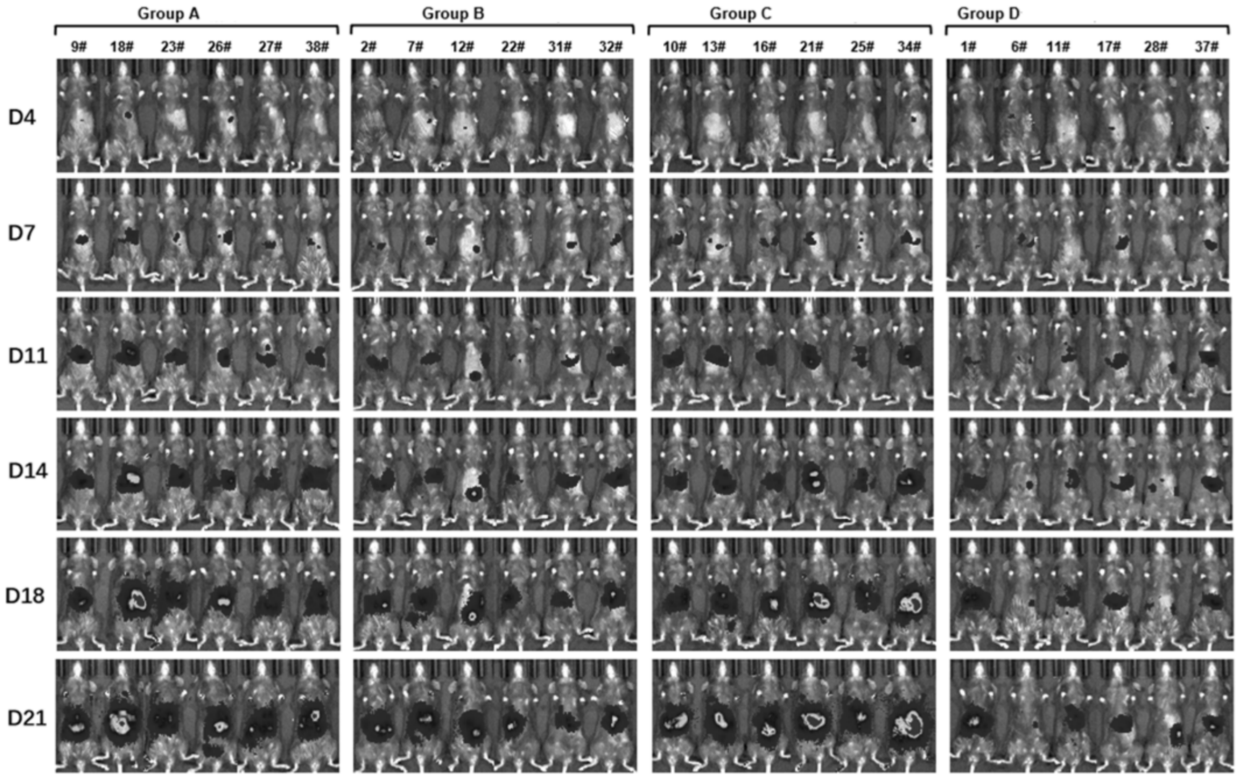


FIG. 22

| CD3 | CD8 | CD45 | CD44 | CD62L |
|---|---|---|---|---|
| APC | PE | APC-cy7 | BV711 | BV421 |

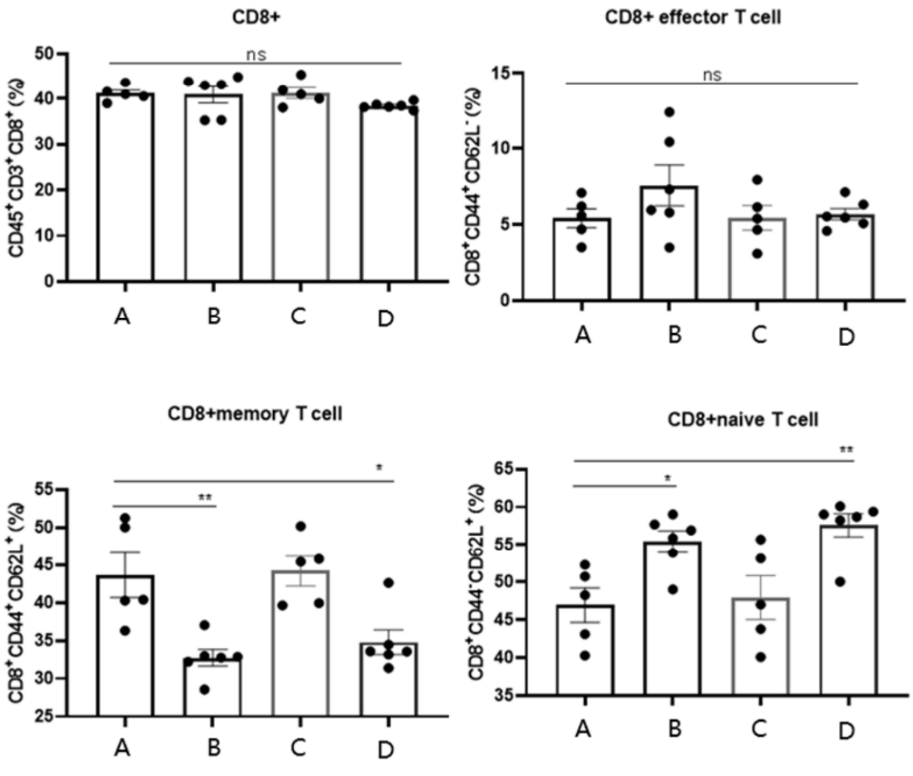


FIG. 23

| CD45 | CD11b | F4/80 | CD64 | CD68 |
|---|---|---|---|---|
| APC-cy7 | BV711 | APC | FITC | PE |

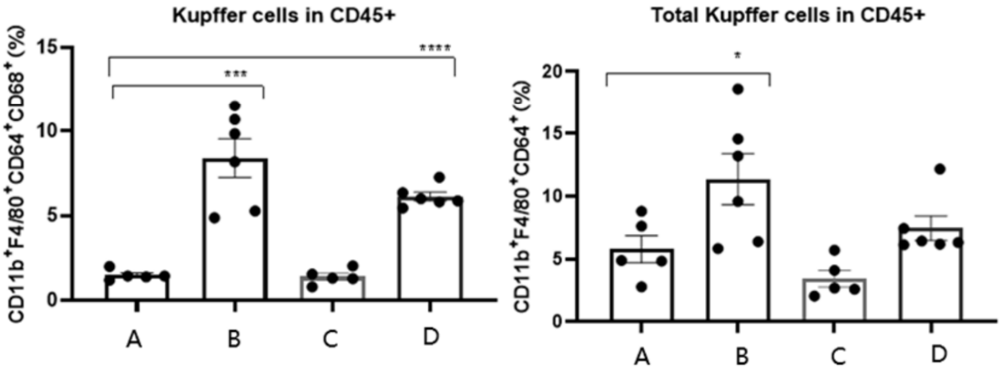


FIG. 24

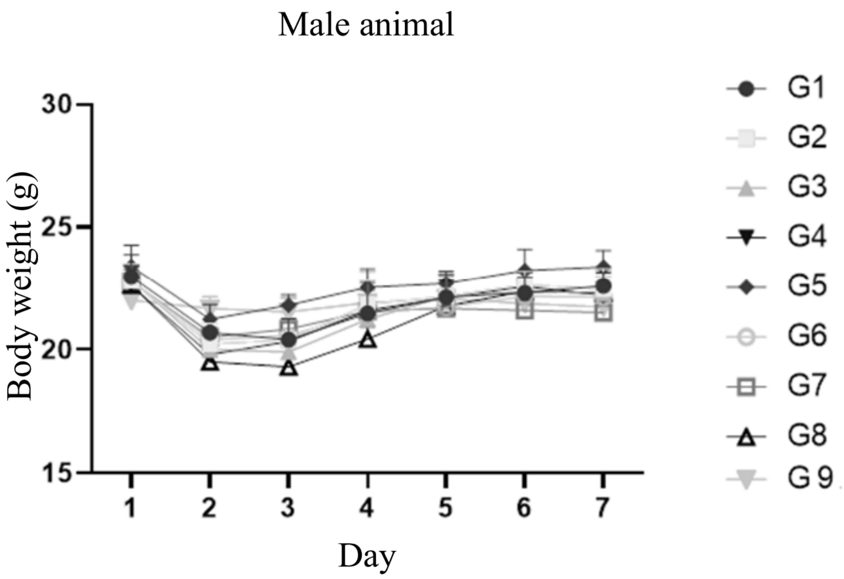


FIG. 25

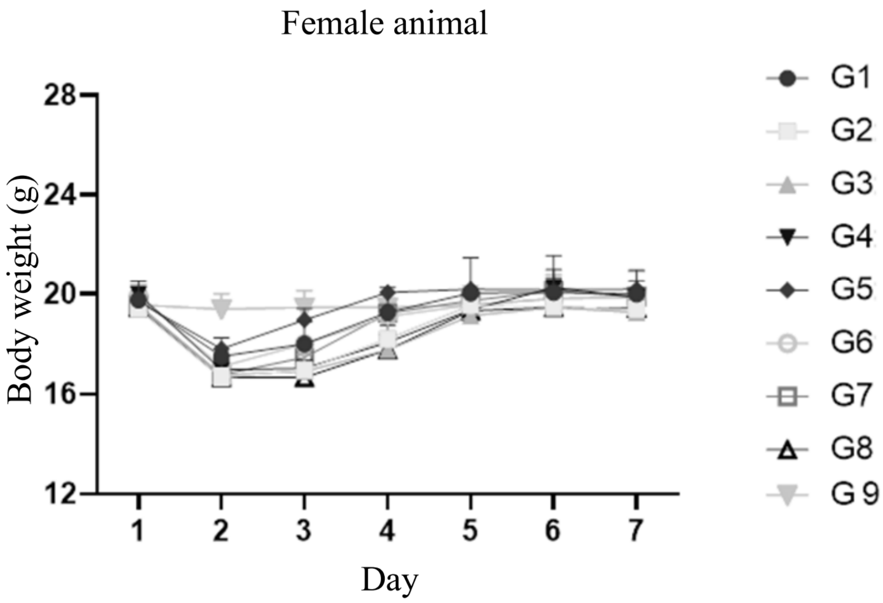
Female animal
Body weight (g)
28
24
20
16
12
1
2
3
4
5
6
7
Day
G1
G2
G3
G4
G5
G6
G7
G8
G 9

FIG. 26

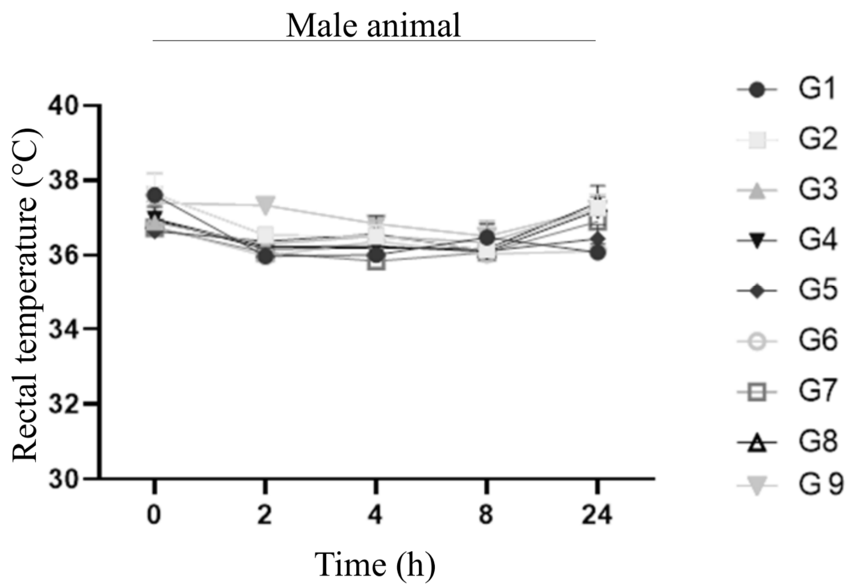
Male animal
Rectal temperature (°C)
40
38
36
34
32
30
0
2
4
8
24
Time (h)
G1
G2
G3
G4
G5
G6
G7
G8
G 9

FIG. 27

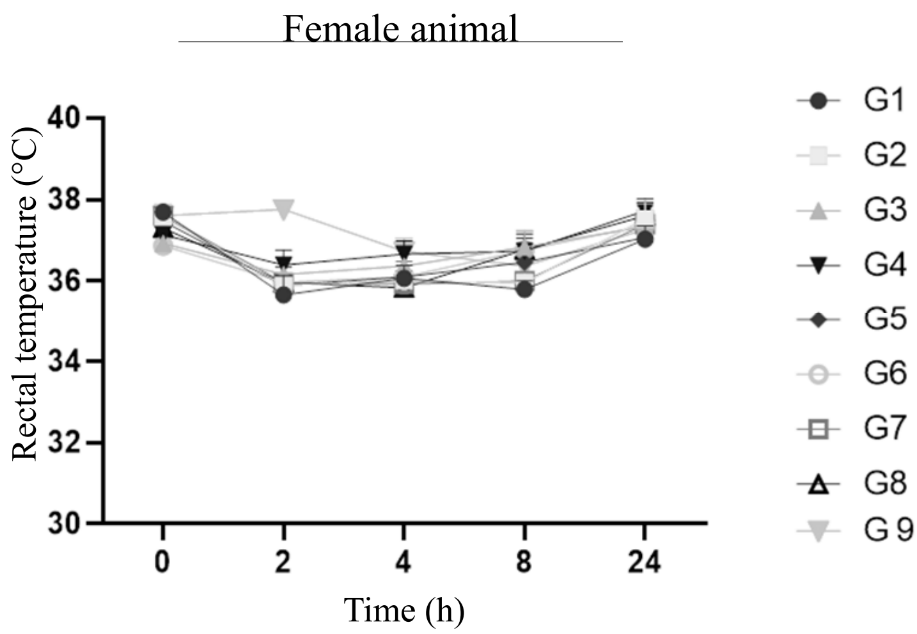
Female animal
Rectal temperature (°C)
40
38
36
34
32
30
0
2
4
8
24
Time (h)
G1
G2
G3
G4
G5
G6
G7
G8
G 9

FIG. 28

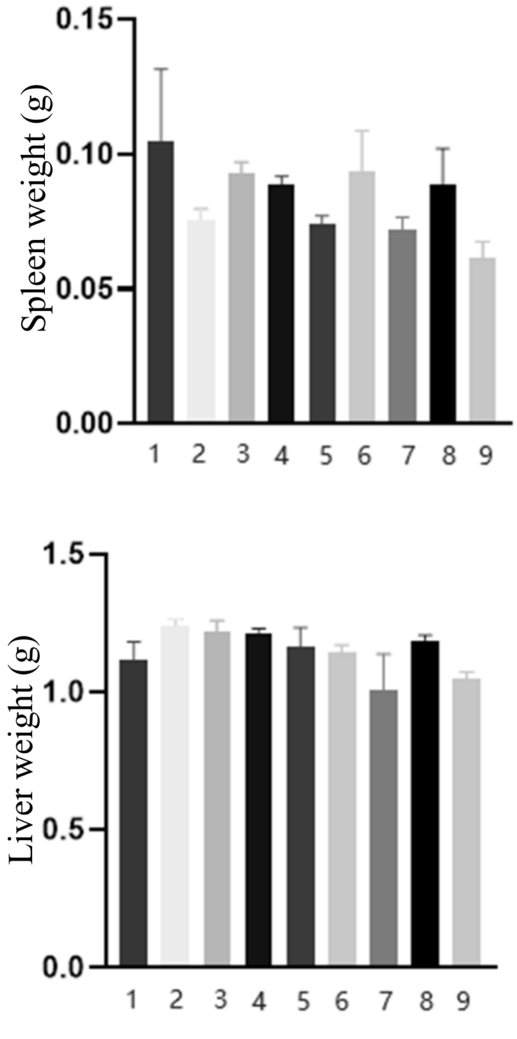
Spleen weight (g)
0.15
0.10
0.05
0.00
1
2
3
4
5
6
7
8
9
Liver weight (g)
1.5
1.0
0.5
0.0
1
2
3
4
5
6
7
8
9

FIG. 29

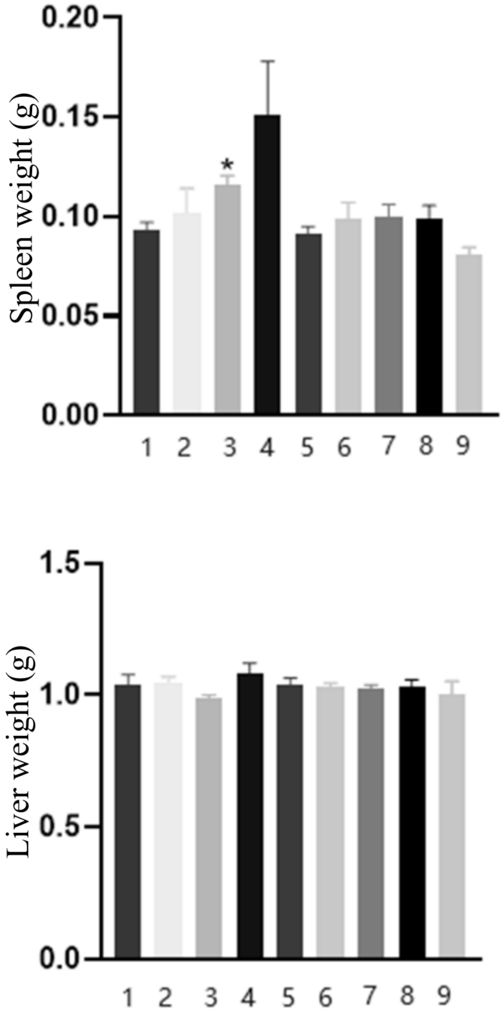
Spleen weight (g)
0.20
0.15
0.10
0.05
0.00
*
1 2 3 4 5 6 7 8 9
Liver weight (g)
1.5
1.0
0.5
0.0
1 2 3 4 5 6 7 8 9

FIG. 30

**INTERNATIONAL SEARCH REPORT**

International application No.
**PCT/CN2024/111818**

**A. CLASSIFICATION OF SUBJECT MATTER**

A61K 31/519(2006.01)i； A61K31/5377 (2006.01)i； A61P31/12(2006.01)i； A61P35/00(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, CNABS, STNext, DWPI, WPABS, WPABSC, ENTXTC, ENTXT, CJFD, CNKI, BAIDU, EMBASE, MEDLINE: 肝脏, 肝炎, 肿瘤, 肝癌, 乙肝, TLR7/8激动剂, Toll样受体, Liver, Hepatitis, Tumor, HBV, Liver cancer, Hepatitis B, PD-1, PD-L1, TLR7/8 agonist, Toll-like Receptor, 结构式检索, structural formula search, 2649170-17-0, 2649170-19-2

**C. DOCUMENTS CONSIDERED TO BE RELEVANT**

| Category* | Citation of document, with indication, where appropriate, of the relevant passages | Relevant to claim No. |
|---|---|---|
| A | CN 115607550 A (TSINGHUA UNIVERSITY) 17 January 2023 (2023-01-17)<br>claims 8 and 9, and description, paragraphs [0002]-[0004] and [0044]-[0046] | 1-15 |
| A | CN 108069963 A (TSINGHUA UNIVERSITY) 25 May 2018 (2018-05-25)<br>claims 5 and 15, and description, paragraphs [0002], [0003], and [0018]-[0025] | 1-15 |
| A | CN 115337406 A (TSINGHUA UNIVERSITY et al.) 15 November 2022 (2022-11-15)<br>claims 49 and 52, and description, paragraphs [0002], [0003], and [0219]-[0225] | 1-15 |
| A | CN 115611945 A (BEIJING YUFAN BIOTECHNOLOGIES CO., LTD.) 17 January 2023 (2023-01-17)<br>claims 7 and 10, and description, paragraphs [0002]-[0004] | 1-15 |
| A | WANG, Zhisong et al. "Structure-Based Design of Highly Potent Toll-like Receptor 7/8 Dual Agonists for Cancer Immunotherapy"<br>*Journal of Medicinal Chemistry*, Vol. 64, No. 11, 28 May 2021 (2021-05-28), ISSN: 0022-2623,<br>pages 7507-7532 | 1-15 |

☑ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **25 September 2024** | **12 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/111818

C. DOCUMENTS CONSIDERED TO BE RELEVANT

| Category* | Citation of document, with indication, where appropriate, of the relevant passages | Relevant to claim No. |
|---|---|---|
| A | HE, Lei et al. "Immune Modulating Antibody-Drug Conjugate (IM-ADC) for Cancer Immunotherapy" *Journal of Medicinal Chemistry,* Vol. 64, No. 21, 03 November 2021 (2021-11-03), ISSN: 0022-2623, pages 15716-15726 | 1-15 |
| A | DAVID, M. et al. "Novel Pyrimidine Toll-like Receptor 7 and 8 Dual Agonists to Treat Hepatitis B Virus" *Journal of Medicinal Chemistry,* Vol. 59, No. 17, 11 August 2016 (2016-08-11), ISSN: 0022-2623, pages 7936-7949 | 1-15 |
| A | 王晓月 等 (WANG, Xiaoyue et al.). "Toll 样受体7 的研究进展 (Non-official translation: Research Progress of Toll-like receptor 7)" *细胞与分子免疫学杂志 (Chinese Journal of Cellular and Molecular Immunology),* Vol. 32, No. 9, 31 December 2016 (2016-12-31), pages 1267-1271 | 1-15 |

Form PCT/ISA/210 (second sheet) (July 2022)

**INTERNATIONAL SEARCH REPORT**
**Information on patent family members**

International application No.
**PCT/CN2024/111818**

| Patent document cited in search report | | | Publication date (day/month/year) | Patent family member(s) | | | Publication date (day/month/year) |
|---|---|---|---|---|---|---|---|
| CN | 115607550 | A | 17 January 2023 | None | | | |
| CN | 108069963 | A | 25 May 2018 | WO | 2019095455 | A1 | 23 May 2019 |
| CN | 115337406 | A | 15 November 2022 | US | 2024252670 | A1 | 01 August 2024 |
| | | | | JP | 2024517341 | A | 19 April 2024 |
| | | | | EP | 4331616 | A1 | 06 March 2024 |
| | | | | WO | 2022237884 | A1 | 17 November 2022 |
| CN | 115611945 | A | 17 January 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 108069963 B